# EUROPEAN PATENT SPECIFICATION

(11) **EP 0 472 593 B1**
(45) Date of publication and mention of the grant of the patent: **14.08.1996**
(21) Application number: 90907698.6
(22) Date of filing: 04.05.1990
(51) Int. Cl.: G01N 33/542, G01N 33/58, C07K 14/00, C12N 1/21, C12N 15/70, C12N 9/38

(54) **METHOD FOR PROTEIN BINDING ENZYME COMPLEMENTATION ASSAYS**
VERFAHREN ZUR PROTEINBINDUNG-ENZYM-KOMPLEMENTATIONSTESTS
METHODE D'ANALYSE DE COMPLEMENTARISATION D'ENZYMES FIXATRICES DE PROTEINE

(30) Priority: 05.05.1989 US 347679
(43) Date of publication of application: 04.03.1992
(73) Proprietor: MICROGENICS CORPORATION, Concord California 94520 (US)
(72) Inventor: HENDERSON, Daniel, R., Benicia, CA 94510 (US)
(74) Representative: Harrison, David Christopher
(86) International application number: US9002491
(87) International publication number: WO9013569

(56) References cited:
- WO-A-86/02666
- US-A- 4 378 428
- US-A- 4 461 829
- US-A- 4 708 929
- CLINICAL CHEMISTRY, vol. 32, no. 9, 1986, Winston, NC (US); D. R. HENDERSON et
- al., pp. 1637-1641/

## Description

The technical field of this invention is enzyme immunoassays.

The prior art teaches many immunoassays based on the pioneering development of radioimmunoassay (RIA) by Yalow and Berson, 1960, J. Clin. Invest., 39:1157. RIAs are characterized by competing fixed amounts of radio-labeled analytes with unknown quantities of unlabeled analytes for fixed amounts of specific antibody. The amount of radioactive analyte either bound to antibody or free in solution is quantitated in an appropriate counter and the concentration of nonradioactive analyte determined. Improvements on this general scheme have included: (1) substitution of the radioactive tracer with enzyme or fluorescent tracers, (2) substitution of polyclonal animal antibodies with monoclonal antibodies, (3) improved methods of signal detection including spectrophotometers, fluorometers, fluorescence polarizers and particle counters, and (4) the introduction of homogeneous assays not requiring physical separation of bound tracer from free tracer. Separation of bound tracer from free tracer frequently requires solid supports such as plastic, paper, glass or acrylamide. Customarily, antibody is bound to the solid phase whereas tracers and unknowns are free in solution. The bound/free separation is accomplished by one or more washes of the solid phase. The residual bound activity is then measured. These assays are known collectively as heterogeneous immunoassays. In comparison, homogeneous assays obviate the need for the imprecise and time-consuming separation steps.

Commercialization of immunoassays has seen a shift in usage from radioimmunoassays, to enzyme-linked immunosorbent assays (ELISA), to homogeneous assays. This shift is due to the commercial demands of speed, simplicity, automation and absence of radioactivity. Homogeneous assays consist of several types: (1) nephelometry, (2) particle counting, (3) fluorescent quenching, (4) fluorescence polarization, and (5) enzyme assays.

The first nephelometer to measure light dispersion to quantitate immune reactions was devised in the late 1960s. These early nephelometers were improved ten years later with new chemistries, lower angles for measuring dispersion angles and the ability to measure the rate of the antigen-antibody reaction during the first seconds after mixing the reactants (Ritchie, Alper and Graves 1969, Arthritis Rheum. 12:693; Deaton et al., 1976, Clin. Chem. 22:1465). These assays are of extremely poor sensitivity and are applicable to determinations of analytes at concentrations greater than 10⁻⁸M, e.g., serum IgE, IgA and IgM levels. In homogeneous particle counting assays, polystyrene particles 0.8 µm in diameter (latex particles) are coated by antibodies. Antigen concentrations can be determined by the concentration of latex particles agglutinated as determined by an instrument capable of distinguishing agglutinated versus nonagglutinated particles (Cambiaso et al., 1977, J. Immunol. Meth. 18:33). Homogeneous fluorescent quenching assays label either antigens or antibodies with a fluorophor. Analyte-antibodyfluorophor complexes yield significantly less fluorescence compared to the antigen-fluorophor or antibody-fluorophor alone (Ullman et al., 1979, J. Biol. Chem. 251:4172; U.S. Pat. Nos. 3,998,943; 3,996,345; 4,174,384; 4,161,515; 4,208,479 and 4,160,016). All these assays involve various methods of quenching fluorescence such that the amount of quenching is related to the amount of the unknown analyte or antibody in the sample. These assays are of low sensitivity (analytes at fluid concentrations greater than 10⁻¹⁰M). The low sensitivity is due to endogenous serum fluorescence and the use of fluorescence in a static non-enzymatically amplified manner. Fluorescence polarization assays are based on the free rotation of antigen-fluorophor in solution which is significantly reduced by antibody binding to the antigen-fluorophor and have found considerable commercial success with low molecular weight (under 1000 daltons molecular weight) analytes (Dandliker et al., 1973, Immunochemistry 10:219).

The various immunoassay methods each possess commercial advantages and disadvantages. RIAs are sensitive and easy to set-up but require radioactivity, separation steps and expensive instrumentation. Heterogeneous assays with enzymes or fluorophores eliminate radioactivity and some instrumentation but require separation steps. From a commercial viewpoint it is desirable to eliminate separation steps for several reasons. Separations (1) are labor intensive, (2) are time consuming, (3) require additional equipment, (4) increase variability in results, and (5) preclude high levels of automation. Despite the many commercial advantages of homogeneous immunoassays only three systems, the enzyme-labeled system of Rubenstein et al., U.S. Pat. No. 3,817,837, the substrate-labeled system of Burd et al., 1977, Clin. Chem. 23:1402, and fluorescence polarization (Dandliker et al., 1973, Immunochemistry) have found commercial success. Yet these three assay systems are limited to small (less than 1000) molecular weight analytes and analytes found in concentrations greater than 10⁻¹⁰M.

Enzyme immunoassays have been a very successful type of homogeneous immunoassay. Several variants of homogeneous enzyme immunoassays have found commercial success (1) the enzyme labeled analyte system; and (2) the substrate labeled analyte system. In the enzyme labeled system the enzymatic activity of the label is decreased when specific antibody binds the analyte-enzyme complex. Analyte to be measured competes with a fixed amount of specific antibody for a fixed amount of the analyte. Enzyme activity is directly proportional to the unknown analyte concentration. The following patents have been issued based on this immunoassay system: U.S. Pat. Nos. 3,817,837; 3,852,157; 3,875,011; 3,966,556; 3,905,871;4,065,354; 4,043,872; 4,040,907; 4,039,385; 4,046,636; 4,067,774; 4,191,613 and 4,171,244. Commercialization of this technology has been limited to low molecular weight analytes and low sensitivity (analytes smaller than 1000 daltons MW at concentrations greater than analytes 10⁻¹⁰M).

The substrate-labeled fluorescent immunoassay involves covalent coupling of the analyte to a fluorogenic substrate for an enzyme. This analytesubstrate conjugate is not fluorescent. In the absence of antibody the analyte-fluorogenic substrate is hydrolyzed by an enzyme yielding a fluorescent molecular species. In the presence of specific antibody, access to the substrate by the enzyme is curtailed yielding decreased fluorescence (Burd et al., 1977, Clin. Chem. 23:1402; Burd et al., Anal. Biochem. 77:56; and Kohen, Hollander and Boguslaski, 1979, J. Steriod Biochem. 11:161). Commercialization of this assay system has been limited to low molecular weight analytes due to steric considerations, and to analytes at concentrations in fluids greater than 10⁻¹⁰M due to considerations analogous to those for the fluorescence quenching assays described above.

Numerous homogeneous enzyme immunoassays have been described which have encountered limited commercialization.

U.S. Pat. No. 4,134,792 describes an immunoassay technique utilizing an enzyme modulator such as an enzyme inhibitor or an allosteric effector as a label. When specific antibody binds to an enzyme modulator-labeled analyte, the enzyme modulator can no longer inhibit the activity of the enzyme. Thus, competition of the enzyme modulator-labeled analyte by free analyte restores inhibition of the enzyme modulator. Other patents in this field include: U.S. Pat. Nos. 3,935,074; 4,130,462; 4,160,645 and 4,913,983.

U.S. Pat. Nos. 4,213,893 and 4,318,983 describe enzyme-immunoassays employing cofactorapoenzyme systems. In particular, U.S. Pat. No. 4,318,983 issued to Hornby et al. (Mar. 9, 1982) describes a method employing flavin adenine dinucleotide (FAD)-labeled conjugates and apoenzymes with which FAD acts as a prosthetic group. U.S. Pat. No. 4,213,893 issued to Carrico et al. (July 22, 1980) describes specific FAD-labeled conjugates, e.g., FAD-labeled thyroxine, which are suitable for use in the Hornby et al. method. FAD-labeled conjugates are monitored by measuring holoenzyme activity generated by incubation of such conjugate with an apoenzyme that requires FAD for catalytic activity. An analyte is covalently coupled to FAD such that the labeled cofactor retains its reactivity with dehydrogenase enzymes. The amount of reduced FAD formed by the dehydrogenase activity is decreased in the presence of antibody specific for the analyte. The fluorometrically monitored appearance of reduced FAD is directly proportional to the amount of analyte (Cohen et al., 1978, in Enzyme-labeled Immunoassay for Hormones and Drugs, S.N. Pal, ed., Walter deGuiter, Berlin and New York, pg. 67-79). A similar system for biotin and 2,4-dinitrofluorobenzene analytes using lactic dehydrogenase and diaphorase has been described (Carrico et al., 1976, Anal. Biochem. 73:271). Both systems suffer from interference from endogenous cofactors and enzymes that are common in serum samples to be analyzed.

Several enzymes have been observed to reform from peptide fragments but only a few regain enzymatic activity including, e.g., ribonuclease A (Richards and Vithayathil, 1959, J. Biol. Chem. 234:1459), staphlococcal nuclease (Light et al., 1974, J. Biol. Chem. 249:2285), and β-galactosidase (Langley and Zabin, 1976, Biochemistry 15:4866). Proteolysis of bovine pancreatic ribonuclease by subtilisin yields two components, a peptide (S-peptide) and a protein (S-protein). Neither S-peptide nor S-protein alone shows appreciable ribonuclease activity. When these components are mixed in molar equivalents, almost the full enzymatic activity is recovered. S-peptide and S-protein re-associate very rapidly and strongly with a K_{eq}=5X10⁻⁹M (Richards and Vithayathil, 1959, supra).. Staphlococcal nuclease shows reconstruction of biologically active enzyme from inactive peptide fragments. Nuclease-T(6-48), including amino acids 6-48 of the full 149 amino acid staphlococcal nuclease structure, re-associates with Nuclease-T(50-149) to form active Nuclease-Tl with a first order rate constant of 0.03-0.05/sec with little temperature variability (Light, supra). As discussed in greater detail, polypeptide fragments (e.g., M15) from deletion mutants of E. coli are known which regain enzymatic activity when combined with small peptide fragments derived from thermally or cyanogen bromide treated β-galactosidase enzyme. One cyanogen bromide-generated fragment is called CNBr2; another is called CNBr24.

More recently, an immunoassay based on the re-association of such polypeptide fragments was described by Farina and Golke (U.S. Pat. No. 4,378,428 issued March 29, 1983) and by Gonelli et al., (1981, Biochem. and Biophys. Res. Commun. 102:917-923). All experimental examples disclosed therein were based on re-association of S-peptide/S-protein to generate ribonuclease catalytic activity. An analyte was covalently attached to a small subtilisin cleavage peptide of ribonuclease, i.e., the S-peptide (amino acids 1-20). This was coupled to an analyte and combined with S-protein (amino acids 21-124) to reform active ribonuclease. Antibody specific for the analyte inhibits the reformation of ribonuclease activity. This assay is limited due to the presence of endogenous ribonuclease activity in all non-autoclaved biological solutions.

Other equally serious faults never addressed by this system include the inability to adjust the equilibrium constant of the associating polypeptides, and an inability to create immunoreactive polypeptides which could couple to large molecular weight proteins while still capable of reforming active enzyme. All polypeptides utilized were non-novel catalytically inactive peptides capable of re-association to form active ribonuclease.

More significant disadvantages with the chemistries proposed by Farina and Golke (U.S. Pat. No. 4,378,428) to attach an analyte to CNBr2 or M15 have been discovered. Attaching an analyte through the available NH₂, COOH, and SH groups on either of the polypeptides have, in all cases tested, yielded polypeptides incapable of complementation. Coupling M15 which has many amino, carboxylic acid and sulfhydryl functionalities, inactivated M15 in all cases, even with carefully controlled conditions. Kinetics indicate a single hit to be sufficient to inactivate activity. CNBr2 contains no internal lysines, a single sulfhydryl group and several carboxylic acid groups. In agreement with Langley (Ph.D. thesis entitled "The Molecular Nature of β-galactosidase α-complementation", UCLA, 1975) coupling to the N-terminal α-amino group inactivates complementation activity of CNBr2. In the preparation of CNBr2 (Langley, Fowler and Zabin, 1975, J. Biol. Chem. 250:2587), the sulfhydryl at position 76 is reduced and alkylated with iodoacetic acid prior to the cyanogen bromide cleavage. If the sulfhydryl is not alkylated CNBr2 activity can be retained early in the steps of purification but is lost prior to purification to homogeneity. Also, if the sulfhydryl is alkylated with a maleimide derivative of an analyte instead of iodoacetic acid, insolubility of the conjugate prevents purification. Finally, in all cases tested, coupling to a COOH moiety of CNBr2 inactivated complementation activity. Therefore, it appears to be difficult to use CNBr2 and M15 to prepare appropriate immunoreactive and complementing reagents.

Mutant polypeptides derived from β-galactosidase are known which can complement or spontaneously restore enzyme activity when added to extracts of appropriate β-galactosidase negative mutants. This phenomenon is known as intracistronic complementation. An example of α-complementation is provided by the M15/CNBr2 complementation system. The M15 mutant polypeptide lacks amino acids 11-41 of β-galactosidase and exists in solution as an enzymatically inactive dimer. A polypeptide derived from β-qalactosidase by cyanogen bromide (CNBr) cleavage, the CNBr2 peptide (CNBr2) , consists of amino acids 3-92. CNBr2, when mixed with the dimer M15, promotes spontaneous reconstruction of the β-galactosidase tetramer with full enzymatic activity (Langley and Zabin, 1976, Biochemistry 15:4866). The M15 peptide is known as an α-acceptor and CNBr2 as an α-donor. While this represents a well-studied complementing system, CNBr2 can serve as α-donor for the M112 dimer, a deletion of amino acids 23-31 within β-galactosidase (Lin, Villarejo and Zabin, 1970, Biochem. Biophys. Res. Common. 40:249; Celeda and Zabin, 1979, Biochem. 18:404; Welphy, Fowler and Zabin, 1981, J. Biol. Chem. 256:6804; Langley et al., 1975, Proc. Natl. Acad. Sci. USA 72:1254). Other α-donors include a polypeptide derived by autoclaving β-galactosidase. This peptide, however, has not been purified and its sequence is unknown. α-acceptors other than M15 and M112 have not been described. In the example of complementation of M15 by CNBr2, amino acid sequences 3-10 and 42-96 are both present in duplicate in the enzymatically active complex.

Intracistronic complementation also occurs at the C-terminus of β-galactosidase (the ω-region). The best known sequence data available is for the X90 ω-acceptor peptide that deletes the last 10 amino acids, 1011-1021. The X90 peptide exists as a monomer and can be complemented CNBr24, a cyanogen bromide digestion product of β-amino acids 990-1023 to reform enzymatically active tetramer (Welphy et al., 1980, Biochem. Biophys. Res. Common. 93:223).

US 4708929 describes complementation assays using two enzyme fragments, e.g. of β-galactosidase, which are combinable in an assay medium to provide active enzyme.

The subject invention provides novel assays for ligands and receptors and compositions for use in the assays comprising enzyme complementation fragments, where the enzyme donor fragment is conjugated to a member of a specific binding pair, where the analyte is cross reactive with the conjugated member of the specific binding pair or is complementary to such conjugated member. The reagents are combined with the sample in an appropriate assay medium and the rate of formation of enzymatic product determined as an indication of the presence of analyte in the medium. The enzyme complementation fragments are modified sequences which provide for the presence of an amino acid having a functionality for linking to the specific binding pair member.

In accordance with the subject invention, diagnostic assays are provided, as well as reagents for use in the diagnostic assays. The reagents comprise two complementary fragments, which when complexed provide for an active β-galactosidase enzyme. Also provided are nucleic acid sequences encoding the mutated fragments and bacteria comprising vectors containing said sequences. Methods for preparing the mutated fragments, including fusion proteins involving amino acid sequences defining epitopes of interest, and methods for expressing such products are disclosed herein.

β-galactosidase is a tetrameric protein having a molecular weight of about 540 kD. The four identical subunits or monomers consist of 1023 amino acids, each with a molecular weight of 116 kD. The monomer may be divided up into two portions, an α-portion of from about 70-100 amino acids of the N-terminus and a remaining portion of the molecule which may partially overlap the α-portion. These two molecules can be used to complement to form a complex which is an active enzyme. For the purposes of the subject invention, the α-region or N-terminal portion will be referred to as the enzyme donor (ED) and the remaining portion as the enzyme acceptor (EA). The enzyme donor will normally be the smaller fragment, except when it serves as a fusion protein. The enzyme acceptor will normally be the larger protein. The ED will be the site of conjugation.

The natural sequence of β-galactosidase may be changed by insertions, deletions, substitutions, and combinations thereof. For the purposes of the present invention at least two substitutions will be employed. For the most part, a substitution will involve exchanging one amino acid for a different amino acid which has a functionality, such as sulfhydryl, amino, hydroxyl, or carboxyl, which allows for conjugation at that site. Two of the substitutions will be to a cysteine or lysine and will be at the 68 and 23 positions.

The basic sequence which will be referred to is as follows:

1           *  5                    10                   15
M  D  P  S  G  N  P  Y  G  I  D  P  T  Q  S

               20          *     25*                  30
S  P  G  N  I  D  P  R  A  S  S  N  S  L  A
                                       6

            *  35                *  40* *  *  *  *  45*
V  V  L  Q  R  R  D  W  E  N  P  G  V  T  Q
                                    20

      *        50       *        55*                  60
L  N  R  L  A  A  H  P  P  F  A  S  W  R  N
                        30

*              65       *        70                   75
S  E  E  A  R  T  D  R  P  S  Q  Q  L  R  S
   40                                 50

               80                85 *        89
L  N  G  L  E  S  R  S  A  G  M  P  L  G
      56

Numbers underneath letters indicate the wild-type
β-galactosidase numbering.

*  Indicates possible sites of amino acid substitutions to
C (Cysteine) or K (Lysine).

Preferred regions for substitutions include the region from about amino acid 1 to amino acid 30; from amino acid 35 to amino acid 45; from amino acid 60 to amino acid 89. It is preferred the substitutions be separated by at least about 5 amino acids, preferably at least about 10 amino acids, and more preferably from about 20 to 60 amino acids. Preferably, the region from about amino acids 48 to 61 are not used for substitution, although the particular site for substitution will to a significant degree depend upon the nature of the conjugate. Thus, one site may be favored over another site when preparing one conjugate as compared to another conjugate.

Sites of particular interest include amino acids 23, 25, 39, 42, 45, 61 and 68. Regions for deletion include the region from amino acids 1 to 20, particularly 5 to 20, or any sequence therein. Regions of interest for substitution of other than a conjugation site include the region from about 70 to 85, particularly from about 72 to 80, more particularly 74 to 77, where a greater or lesser number of amino acids may be introduced, where the substitutions may be conservative or non-conservative. By conservative is intended having the same or substantially the same charge type and general conformation, for example, neutral amino acids may be substituted for other neutral amino acids, aromatic amino acids for other aromatic amino acids, charged amino acids for other charged amino acids of the same charge type, and the like. Furthermore, one could consider for conservative changes, retaining a hydrophobic region as compared to a hydrophilic region, where non-conservative would be to change the nature of the region from hydrophilic to hydrophobic or vice versa.

A large number of linking groups may be employed for joining a wide variety of specific binding pair members to a functionality present in the ED. As already indicated, for the most part, the functionality present on the ED for linking will be a mercaptan or amino group. For mercaptans, of particular interest are a wide variety of readily available reagents, involving activated halogen, activated olefin, or mercapto, where the first two form thioethers and the second a disulfide. Specific compounds include N-maleimidobenzoic acid, α-bromoacetamidocyclohexanecarboxylic acid, N-maleimidosuccinic acid, methyldithioacetic acid, etc. For amino groups, a wide variety of active halogens or carboxylic acid groups may be employed, particularly activated carboxylic acid groups, where the carboxylic acid groups may be activated with carbodiimide, active esters, such as N-hydroxy succinimide, o-nitrophenol, p-nitrophenol, etc. The procedures for conjugation are well known in the literature and are amply illustrated by U.S. Patent Nos. 3,817,837; 4,262,089; 4,233,401; 4,220,722 and 4,374,925.

The linking group may merely be a bond, for example where the ligand has a carboxylic acid group which can be activated to react with the amino group of ED or may be of one or more atoms other than hydrogen, usually from about 1 to 24 atoms, more usually from about 1 to 12 atoms. Besides carbon atoms, the atoms in the chain may include nitrogen, sulfur, oxygen or the like.

Besides conjugation through a chemical reaction, one can provide for a fused protein by preparing a nucleic acid sequence encoding the ED joined to an amino acid sequence which is immunologically cross-reactive with a peptide of interest. One can synthesize appropriate strands of deoxynucleotides which provide for a fusion protein of the epitope(s) of interest with the ED, where the epitope(s) of interest may be at the N- or C- terminus of the ED, preferably the N-terminus.

The fusion protein may be of any size, usually being not greater than about 500 amino acids, more usually being not greater than about 200 amino acids, and preferably not greater than about 150 amino acids, including the ED sequence.

### Enzyme Acceptors

The enzyme acceptor may be naturally occurring or synthetic. By synthetic is intended the use of recombinant DNA techniques to provide for the desired amino acid sequence. For the most part, the sequence of M15 will be employed as the basic sequence for the enzyme acceptor (EA). Of particular interest is the reduction in the number of available sulfhydryl groups present in the sequence. The EA M15 appears to have 5 cysteine residues available on the surface. Some EAs may have fewer than 5 cysteine residues as a result of substitutions of the cysteine, particularly conservative substitutions, such as G, A, M, S, T, etc.

### Analytes

The analyte may be any member of a specific binding pair, which comprises ligands and receptors where a complementary member of a pair has a high affinity for the other member of the pair, usually at least about 10⁻⁶/mole. The ligands will be at least of about 125D (Dalton) and usually higher, more usually at least about 150D, and may be 500,000D or more. For the most part, the ligands will be less than 200kD, more usually less than 100kD. In many cases, where the ligand is of high molecular weight, greater than 50kD, a fragment of the ligand which is immunologically cross-reactive with the ligand may be employed in the conjugate.

A large number of ligands are listed in U.S. Patent No. 3,996,345. Ligands for the most part will be drugs, drug metabolites, biologically active molecules, such as steroids, vitamins, proteins, receptors, lymphokines growth factors, etc., industrial pollutants, pesticides and their metabolites, herbicides and their metabolites, flavoring agents, food poisons, components of pathogens, toxins, as well as any other substance of interest.

Receptor analytes may be any protein, nucleic acid or saccharide, which arbitrarily is chosen as the receptor, usually having a cleft or surface concavity where the ligand binds. Receptors for the most part are immunoglobulins, surface membrane proteins, which include T-cell receptors, MHC antigens, blood proteins, such as thyroxine binding globulin, lipoproteins, etc., enzymes, avidin, and the like.

Thus, any compound for which a complementary binding member can be found or prepared may be determined by the subject assays. The analyte need not be a single compound, but an aggregation of compounds such as may be found in microorganisms, such as viruses and bacteria, membrane fragments, or other aggregation or complex organization of molecules.

### Method of Preparation

The subject polypeptide sequences may be prepared by any convenient means. Thus, the sequences may be synthesized on commercially available synthesizers. However, where the sequence is to be greater than about 50 amino acids, the efficiency of synthesis drops, so that other methods may become more attractive. One of the alternative methods is the use of recombinant technology, where single strand deoxyonucleotide sequences are prepared encoding portions of the sequence of interest or sequence complementary thereto. The strands are for the most part overlapping, so that when hybridized and ligated, the resulting double stranded DNA sequence encodes the desired amino acid sequence. The sequence may then be inserted in any convenient expression vector. A large number of expression vectors are commercially available or have been described in the literature. While for the most part prokaryotic hosts will be employed, in some instances eukaryotic hosts will be desirable, particularly where there are fusion proteins and it is desired that the fusion protein be processed. The vector will normally comprise the coding sequence, 5' in the direction of transcription to the coding sequence, a transcriptional initiation regulatory region or promoter, and 3' to the coding region in the direction of transcription, a transcription and translation termination regulatory region, so as to provide an expression cassette. Particularly, for transformation into prokaryotes, there will be a replication system which is functional in the host and provides for stable maintenance of the vector. A wide variety of replication systems have been identified and used in prokaryotes, as well as eukaryotes. Also, there will normally be a marker for selection of those host cells which have been transformed with the vector. For the most part, the marker will be resistance to a toxin, e.g., an antibiotic or provide for complementation of an auxotrophic host to provide prototrophy.

Transformation may be achieved by transfection, using a viral vector, protoplast fusion, transformation using calcium precipitated DNA, or other convenient technique. The manners of transformation are conventional and may be found in Maniatis et al, Molecular Cloning: a Laboratory Manual, Coldspring Harbor Laboratory, Coldspring Harbor, NY 1982.

If desired, the sequence may include a signal sequence for secretion of the polypeptide product from the host. A wide variety of signal sequences are available, particularly for eukaryotic organisms. Where a signal sequence is not employed, it will be necesary to lyse the cells in order to extract the desired polypeptide.

The transformed host cells may be grown in an appropriate medium for sufficient time for the desired polypeptide to be formed and the product isolated, the manner depending upon whether the product was secreted or retained in the cytoplasm. Once the product is isolated, it may be purified in conventional ways, by chromatography, electrophoresis, gradient density separation, or the like.

The enzyme acceptor may be prepared in the same way or may be isolated from the host that produces the M15 sequence naturally. The particular manner in which the enzyme acceptor is produced is not critical to this invention.

Once the fragments are obtained, they may be modified as previously described. In the case of the enzyme acceptor, sulfhydryl groups may be capped or otherwise modified as appropriate.

### Assay

The protocols for the assay may be varied widely, depending upon whether a manual or automatic system is being employed, the sensitivity of the assay, the speed with which the assay is to be carried out, the nature of the analyte, and the like. The assay may be competitive or non-competitive, again depending upon the nature of the analyte. The various components of the assay may be added sequentially or concomitantly. The sample may be subject to prior preparation or may be used as obtained.

For the most part, the assay medium will be buffered at a pH in the range of about 6 to 8, with a convenient buffer, such as phosphate buffered saline, tris, or the like. The significant factor is that the buffer does not inhibit the enzyme reaction. The ionic strength is not critical. The temperature for the assay will usually be about 20°C, preferably elevated, but below 60°C, preferably below about 40°C. The assays are performed at atmospheric pressure.

The concentration of the enzyme donor conjugate in the assay medium will usually be in the range of about 1nM to about 60nM, more usually about 5nM to 50nM, preferably about 10nM to 25nM. The enzyme acceptor will usually be in substantial molar excess, usually at least about 1.5 molar excess, preferably at least about 5 molar excess. The molar ratios of enzyme donor conjugate to enzyme acceptor conveniently are in the ratio of about 1:30 to 1:80, more usually 1:50 to 1:60. The concentration of the enzyme donor conjugate will usually exceed the highest concentration of the analyte anticipated to be encountered in the sample.

Where ligand is present in the conjugate, the optimal ratio of ED-analyte conjugate and anti-analyte antibody will be determined in the presence of EA so as to span the dynamic range of the assay and also to minimize the background activity. The response of the enzyme-catalyzed rate to analyte concentration in relation to background level is optimized.

The ratio of the concentration of the ED-analyte conjugate and anti-analyte antibody will be such as to substantially achieve minimum enzyme rate under assay conditions in the absence of ligand analyte, while maintaining linearity of the rate varying with analyte concentration over the desired assay range. Usually the concentrations of antibody and conjugate will be within at least about 85%, more usually within at least about 95% of the concentrations necessary to optimize conditions.

Varying amounts of sample can be used, depending upon the concentration of the analyte, the nature of the sample, and the sensitivity of the assay When the assay is serum, usually the sample will comprise from about 1% to 10% of the volume of the assay medium.

An enzyme substrate is employed that when cleaved by the enzyme results in a change in the amount of light absorbance (optical density) or emission of the assay medium. That is, cleavage of the substrate results in the appearance or disappearance of a colored or fluorescent product. Preferred enzyme substrates include o-nitrophenyl galactoside (ONPG) and chlorophenyl red-β-galactoside (CPRG). ONPG, CPRG and other comparable enzyme substrates are commercially available. ONPG will generally be used in a concentration of from about 0.5 to 2.0 mg/ml. Other substrates will be used in concentrations to provide a comparable signal to ONPG.

The sample and conjugate are combined in an appropriate assay medium. The EA may be present or may be added subsequently. The complementary member to the specific binding pair member of the conjugate may be present as the analyte in the sample or may be added as a reagent, normally being present not later than the combining of the ED conjugate and EA. However, one may reverse the order and allow for formation of the enzyme, followed by addition of the complementary specific binding pair member and observe the change in enzyme activity with time. The enzyme substrate may be added any time, but will usually be added after incubation of the various components of the assay.

Usually, one or more readings will be taken after incubation, the interval varying from about 30 sec. to about 20 min., usually from about 1 to 10 min. between the readings. The time for the first reading based on the addition of enzyme substrate will generally be from 30 sec. to about 10 min., more usually within about 5 min. While a single reading may be taken, it will usually be desirable to take more than one reading, so that common errors may be cancelled out.

Desirably, standard solutions will be prepared of known concentrations of analyte to serve as standards for comparison with the sample. In this way, accurate quantitative determinations may be obtained.

The following examples are offered by way of illustration of protocols by which a range of enzyme donor and enzyme acceptor molecules, including molecules according to the present invention, may be obtained, tested and used in e.g. diagnostic assays. Of the enzyme donor molecules specifically mentioned, ED 28 with substitutions to cys at positions 1 and 46 (wild-type numbering) is a donor according to the present invention.

### EXPERIMENTAL

### Enzyme-Donors

### p125 Enzyme-Donor

The plasmid p125 was genetically engineered to place an α-donor sequence under regulatory control of a temperature inducible promoter (λPr). In addition, the expressed α-donor peptide contains a unique cysteine residue near the C-terminal end. This was accomplished by cleaving the plasmid pUC13 with BglI and the resultant single-stranded termini were removed by treatment with Sl nuclease. The plasmid was then digested with BamHI. The approximately 170 bp DNA fragment encoding the β-galactosidase α-gene was then purified by agarose gel electrophoresis. (See U.S. Patent No. 4,708, 929, Fig. 2, in referring to figures in the subsequent description, the figures of the indicated patent are intended.)

Plasmid pβa12 is a derivative of plasmid pCVQ2 (Queen, 1983, J. Molec. Applied Genetics 2:1) which carries the lac operon under regulatory control of the temperature inducible λPr promotor. To make the λ regulatory sequences available for other genetic constructions the plasmid pβga12 was modified. Plasmid pβga12 was digested with BamHI and SalI and the DNA sequences encoding the lac operon were removed. The DNA fragment containing pBR322 sequences (including amp^{r} and ori) and λCI were isolated by agarose gel electrophoresis. Synthetic DNA linkers containing recognition sequences for BamHI, EcoRI, HindIII, SalI and XbaI were ligated and then cleaved with BamHI and SalI to create shorter multi-linker segments with BamHI and SalI cohesive ends. These DNA fragments were ligated to the BamHI/SalI fragment isolated from pβga12. The resultant plasmid, p121B contains EcoRI and XbaI recognition sites between the BamHI and SalI of the vector. Plasmid p121B was digested with BamHI and PvuII. The BamHI/PvuII DNA fragment containing the 8-lactamase gene (which confers resistance to ampicillin, amp^{r}), the phage λCI gene (a temperature controlled repressor) and the plasmid origin of replication (ori) was purified by agarose gel electrophoresis. The Bg1I(-)/BamHI DNA fragment from pUC13 and the BamHI/PvuII DNA fragment from p121B were ligated using T4 DNA ligase as shown in FIG. 2A. The recombinant plasmid was transformed into JM83, an E. coli bacterial host for growth of the single-stranded phage M13 and its recombinant which encodes the β-galactosidase mutant polypeptide M15 (Messing, 1979, Recombinant DNA Technical Bulletin, NIH Publication No. 79-99, 2, No. 2:43-48) and plasmid p125 was selected, In vivo complementation occurred at 42°C but not at 32°C demonstrating that plasmid pl25 produces a temperature inducible β-galactosidase α-protein.

### H, B, M and P Series Enzyme-Donors

In one series of experiments, to obtain enzyme-donor peptides of the type containing an analyte-coupling domain, various sized α-regions were isolated from pUC13 (Vieira and Messing, 1982, Gene 19:259-268; Messing, 1983, Methods in Enzymology 101:20-78; Bethesda Research Laboratories, Gaithersburg, MD) digested with HaeII, BglI, MstI or PvuI yielding H-series, B-series, M-series and P-series respectively. The B-, P- and H-series were treated with T4 DNA polymerase and S1 nuclease. The M-series were not treated. Each series of DNA was digested with SacI which is located in the multiple cloning site, and the small DNAs encoding an α-complementing peptide were purified by agarose gel purification, electrophoresed onto DEAR-cellulose paper (Schleicher and Schuell, Keene, NH), eluted and ethanol precipitated as described by the manufacturer.

Plasmid p141 which carries an E. coli trp promotor (EcoRI-SstI, 120 bp) cloned in the 2.3 kb EcoRI-PvuII fragment of pBR322, was digested with NdeI and treated with DNA polymerase Klenow fragment and dATP and dTTP (PL Biochemicals, Milwaukee, WI). The resultant DNA was digested with SacI and used as a vector to receive the M, B, H and P series of DNAs. Following treatment with T4 DNA ligase, the DNAs were transformed into E. coli strain E9001 (Δlac pro, thi, supE, F' proAB, lacI^{Q}, Z M15 also referred to as strain 71.18; Messing et al, 1977, Proc. Natl. Acad. Sci. USA 75; 3642-3646). The DNA constructions were sequenced by the method of Maxam and Gilbert (1980, Methods in Enzymology 67:499) and are shown in FIG. 4. Also illustrated (*) are the sites for covalent attachment of an analyte.

The resultant strains encoding α- regions under Trp control in E. coli strain E9001 were for series B, strain MG130 carrying plasmid p130; for series M, strain MG129 carrying plasmid p129; and for series H, strain MG131 carrying plasmid p131.

To improve expression levels of the different cloned α-regions, the α-regions were transferred to new plasmids and placed under control of the λPr operator-promotor. For example, to construct MG141, the gene encoding the DNA sequences of H6 from the H-series was placed under Pr control, by replacement of the Trp promotor for the λPr and λCI genes as described below.

Plasmid p131, containing H6 under the Trp operator-promotor control was digested with EcoRI and the larger, approximately 2.1 kb fragment was isolated by agarose gel electrophoresis. The λPr and λCI genes were gel purified from the small fragment of an EcoRI digestion of p125. The 2.1 kb fragment of p131 was ligated to the small fragment from pl25 in effect replacing the Trp promotor with the λPr and λCI promotor system. This protocol was also repeated with p130 and p129 to yield the following plasmids and strains under λPr control for series B, strain MG139 carrying plasmid p139; for series M, strain MG140 carrying plasmid p140; and for series H, strain MG141 carrying plasmid pH6. The DNA constructions were sequenced by the method of Maxam and Gilbert, Methods in Enzymology 67:499 (1980), and shown in FIG. 4.

### Enzyme-Donor 3

Enzyme-donor 3 (ED3) was constructed from enzyme-donor 1 (ED1) which was constructed from H6. ED1 was constructed as follows:

Synthesis of DNA fragments was performed on an Applied Biosystems, Inc. (ABI, Foster City, Calif.) Model 380A DNA Synthesizer. Each sequence was entered into the program memory and the machine automatically manufactured the desired single strand of DNA, cleaved each fragment from the controlled pore glass support, and collected the DNA in a vial. DNA samples were treated with 1.5 ml of concentrated NH₄OH for 6-24 hours at 55°C and taken to dryness in a Savant Speed Vac Concentrator.

The dried pellet of each DNA fragment was dissolved in a small quantity of formamide (100-200 µ/1) and purified on a 12% acrylamide gel (BRL Model SO, 34-40 cm, 1.6 mm thickness) and was electrophoresed overnight at 200 volts. The desired band was visualized using Baker-flex silica gel 1B-F (J.T. Baker Chemical Co.) as a fluorescent background. The desired DNA band was cut out of the gel with a razor blade and the DNA electrophoresed from the acrylamide gel fragment in an International Biotechnologies, Inc. (IBI) Model UEA unit following the manufacturer's instructions. The DNA was collected in a small volume of buffer and ethanol precipitated. The fragments were treated with T4 polynucleotide kinase according to the manufacturer's instructions. Complementary DNA strands were combined, heated to 90°C for 2 minutes, and slowly cooled to room temperature. The annealed DNAs were purified by agarose gel electrophoresis to remove unhybridized strands and used in ligation reactions.

The starting plasmid was p169 which contains the H6 gene under λPr control inserted between restriction sites BamHI and SalI (see FIG. 11). The change from H6 to ED1 involved changing both the N-terminus and C-terminus of H6 while leaving the α-domain in between intact. Two aliquots of p169 were cut with restriction enzymes. The first aliquot was digested with EcoRI and BglI and the small 150 bp fragment was gel purified. The second aliquot of p169 was digested with BamHI and SalI. This cleaves the plasmid into vector and the α-donor gene region. The vector portion was gel purified.

The new N-terminal coding region of ED1 was a 75 bp DNA fragment synthesized by the Applied Biosystem, Inc. machine (see FIG. 12). The new C-terminal coding region, a 50 bp DNA fragment, was also synthesized (see FIG. 12). The two (2) new DNA fragments were ligated to the small EcoRI-BglI H6 DNA fragment. This mix was cut with BamHI and SalI to yield the new ED gene of about 275 bp. This DNA fragment was gel purified and ligated into the vector Bam-Sal DNA fragment.

After confirming the ED1 sequence, this plasmid (p181, see FIG. 13) was cut with BamHI and EcoRI which removes the 75 bp ED1 N-terminus. This region was replaced by a newly synthesized fragment of 30 bp (see FIG. 12) substituted into the Bam-EcoRI space.

Thus, ED3 is 15 amino acids shorter than ED1 and has a cysteine residue near its N-terminus. ED1 has no cysteine or lysine in its sequence. FIG. 14 depicts the amino acid sequence of ED3.

### Enzyme-Donor 3A

The amino acid sequence of enzyme-donor 3A (ED3A) is shown in FIG. 14. The peptide is synthesized on a Beckman (Palo Alto, Calif.) 990BN Peptide Synthesizer. Methods for synthesis are as described by Stewart and Young (Solid Phase Peptide Synthesis, 176pp, Pierce Chemical Co., Rockford, Ill., 1984). General chemicals are from Aldrich (Milwaukee, Wis.). BOC-amino acids are from Peninsula Laboratories (Belmont, Calif.). Side chain protections are Boc-Thr (OBzl), Boc-Glu (OBzl), Boc-Ser (OBzl), Boc-Asp (OBzl), Cys (MeOBz1), Boc-Asn/HOBT, Boc-Arg (TOS) and Boc-His (TOS). Aminomethylpolystyrene solid phase resin beads from Bio-Rad Laboratories (Richmond, Calif.) are esterified to p-hydroxymethylphenylacetic acid Boc-Thr (OBzl) with dicyclohexyl carbodiimide as described by Stewart and Young (1984). The synthesis scale used is 1 mmole Boc-Thr attached to the solid phase resin and 3 mmoles of each Boc amino acid. The synthesizer is then programmed to carry out the synthesis. The resultant peptide is cleaved from the resin with anhydrous hydrofluoric acid and extracted with acetic acid. Following hydrogenation, the peptide is purified by preparative reverse phase HPLC using a Waters phenyl column with a 0-80% acetonitrile gradient in water containing 0.1% TFA and 0.1% ethane thio. The partially purified peptide is dialyzed exhaustively into 1 mM NH₄HCO₃, 1 mM 2-mercaptoethanol and lyophilyzed. Amino acid analysis of the peptide is shown in Table I.

**TABLE I**

| AMINO ACID ANALYSIS OF ED3A | | |
|---|---|---|
| AMINO ACID | THEORETICAL | FOUND |
| ASP | 5 | 4.24 |
| THR | 3 | 2.13 |
| SER | 3 | 2.39 |
| GLU | 5 | 5.22 |
| PRO | 3 | 3.33 |
| GLY | 1 | 0.87 |
| ALA | 5 | 5.65 |
| CYS-PE | 1 | 1.10 |
| VAL | 3 | 2.27 |
| MET | 0 | 0 |
| ILE | 1 | 0.48 |
| LEU | 4 | 3.12 |
| TYR | 0 | 0 |
| PHE | 1 | 1.16 |
| HIS | 1 | 1.11 |
| TRP | 2 | 1.61 |
| LYS | 0 | 0 |
| ARG | 5 | 5.00 |
| The molecular weight equals 4942.53 with the average molecular weight of an amino acid being 114.943. | | |

In summary, the polypeptides shown in FIG. 4 provide convenient coupling side chains at varying distances from the required α-complementing sequence. The DNA sequences encoding the peptides made by recombinant methods were determined by the standard Maxam and Gilbert technique, confirming the predicted structures. The amino acid composition of H6 was confirmed by amino acid analysis.

### ED Enzyme-Donor Series

A series of enzyme-donors called the ED series was constructed by recombinant DNA techniques. ED3 has already been descsribed. Other members of the series include ED4, ED5, ED7, ED8, ED13, ED14, ED15 and ED17. The amino acid sequences of the ED series of enzyme-donors appear in FIG. 15, A-I.

The gene coding for ED4 was constructed by first synthesizing a DNA fragment on an Applied Biosystems, Inc. Model 380A DNA Synthesizer (as described previously) of the following sequence: 
*                    50
TGC CCT TCC CAA CAG TTG CGC AGC CTG AAT

TA ACG GGA AGG GTT GTC AAC GCG TCG GAC TTA
   PvuI

                    60
GGC CTC GAG TCT AGA TCT GCA GGC ATG (57 mer)

CCG GAG CTC AGA TCT AGA CGT CC (55 mer)
                            SphI
 The "T" marked with an asterisk represents a change from a "C". This fragment was ligated to the BatHI-PvuI piece from plasmid p181 (ED1) (see FIG. 13). The resultant piece was ligated back into the vector (from ED1-p181) having removed the BamHI-SphI region. The C to T change creates a cysteine (cys) residue and destroys the PvuI site after ligation. (The sticky ends remain the same for ligation).

The gene coding for EDS was constructed by first synthesizing a DNA fragment of the following sequence: 
*                  **  40                    45
T TGG CGT AAT TGC GAA GAG GCC CGC ACC GAT (31 mer)

A ACG GCA TTA ACG CTT CTC CGG GCG TGG C (29 mer)
PvuII                                  PvuI
 The "T" marked with an asterisk represents a change from a "C". The "T" marked with a double asterisk represents a change from an "A". The C to T change destroys the PvuII site. The A to T changes a serine residue to cysteine residue. This fragment was ligated to the BamHI-PvuII piece and PvuI-SalI pieces from plasmid p182(ED2 or M15)DNA (see FIG. 13). The ligated material was cut with BamHI and SalI and inserted into p182 with the BamHI-SalI region removed.

The gene coding for ED7 was constructed by cutting p183 (ED3) and p184 (ED4) plasmids (see FIG. 13) with both EcoRI and SalI. The vector from p183 was gel purified (effectively removing the EcoRI-SalI (α) region. In contrast, the small EcoRI-SalI (α) region from p184 was gel purified. The p184 EcoRI-SalI region was then inserted and ligated into the p183 EcoRI-SalI vector.

The gene coding for ED8 was made using site specific mutagenesis in M13mp11 phage DNA. A primer was made (sequence GGT AAC GCA AGG GRT TTC CCA GTC). This primer is complementary to the sense strand of the region coding for amino acids 15-22. The desired change was a G to T in codon 20 which changed a Gly to Cys at amino acid 20 in the α region of the M13mp11 DNA. This was accomplished by hybridizing the primer to single-stranded M13mpll phage DNA and extending the primer using DNA polymerase I "Klenow fragment: and T4 DNA ligase overnight at room temperature. This DNA was treated with S1 nuclease to eliminate non-doublestranded DNA and then transformed into JM103 cells. Phage from this transformation were isolated; the DNA was purified and the primer extension and transformation was repeated a total of 3 times. Each repeat enriched for the desired product. Finally, mini-prep analysis was performed on M13 RF DNA from individual placques. The desired base change eliminated the BstNI site. Restriction analysis of mini-prep DNA with BstNI identified candidates. From the double-stranded M13 RF DNA carrying the desired change, a BamHI-BglI piece was cut out and exchanged for a BamHI-BglI piece in the plasmid coding for ED2.

The gene coding for ED13 (p193, see FIG. 16) was constructed by first synthesizing (as above) a DNA fragment of the following sequence:

Lys
change Eco RI
BamHI
-3 0
GAT CCC AGC GGC GAT CCC CGG GCA AAA TCG (30 mer)
GG TCG CCG CTA GGG GCC CAT TTT AGC TTA A (30 mer)

This synthesized fragment was substituted into p182 (ED2) as described above in constructing ED3.

The gene coding for ED14 (p194, see FIG. 16) was constructed by first synthesizing (as above) a DNA fragment of the following sequence:

Lys
change
50 55
AAA CCT TCC CAA CAG TTG CGC AGC CTG AAT
TA TTT GGA AGG GTT GTC AAC GCG TCG GAC TTA
PvuI
60
GGC CTC GAG TCT AGA TCT GCA GGC ATG (57 mer)
CCG GAG CTC AGA TCT AGA CGT GC (55 mer)
SphI

This synthesized fragment was constructed with the same strategy used for ED4, but resulting in a lysine residue instead of a cysteine substitution.

The gene coding for ED15 (p195, see FIG. 16) was constructed by first synthesizing (as above) a DNA fragment of the following sequence:

Lys
change 40
PvuII 44 PvuI
T TGG CGT ATT AAA GAA GAG GCC CGC ACC GAT (31 mer)
A ACC GCA TTA TTT CTT CTC CGG GCG TGG C (29 mer)

This fragment was inserted into p182 (ED2 or M15) in the same way used to construct ED5.

The gene coding for ED17 (p197, see FIG. 16) is a combination of the ED13 and ED14 genes, constructed in the same way as the gene coding for ED7 was.

The following is a listing of the enzyme acceptors which may be used with the ED series of enzyme donors.

| ENZYME DONOR | ENZYME ACCEPTOR* |
|---|---|
| ED3 | M15,EA1,EA14,EA20,EA22 |
| DE4 | M15,EA1,EA14,EA20,EA22 |
| ED5 | M15,EA1,EA14,EA20,EA22 |
| ED7 | M15,EA1,EA14,EA20,EA22 |
| ED8 | M15,EA1,EA14,EA20,EA22 |
| ED13 | M15,EA1,EA14,EA20,EA22 |
| ED14 | M15,EA1,EA14,EA20,EA22 |
| ED15 | M15,EA1,EA14,EA20,EA22 |
| ED17 | M15,EA1,EA14,EA20,EA22 |

| | |
|---|---|
| *Other enzyme-acceptors have not been tested. | |

### Enzyme-Acceptors

In one group of experiments, a series of in-frame sequence deletions of the β-qalactosidase gene were constructed to prepare a series of enzyme-acceptors according to methods described above. pUC13 was digested with PvuII (yielding a blunt end) and ligated to an 8 bp synthetic DNA linker containing an XhoI restriction site to create a new plasmid, pUC13X.

The α-region containing the XhoI restriction site was then replaced into the entire lacZ gene, which encodes native β-galactosidase without disrupting the remainder of the lacZ gene or the background plasmid. The Z gene contains two BglI sites. The first of these BglI sites is contained within the α-region in pUC13 downstream from the PvuII site where the XhoI linker was inserted. Thus the α-region from pUC13X was removed from the rest of the plasmid by digesting with BamHI and BglI and the 170 bp fragment designated B1X.

The remainder of the lacZ gene which encodes β-galactosidase was obtained from the plasmid pβga12 (Queen, 1983, J. Mol. Appl. Genet. 2:1). This plasmid was digested with BglI and EcoRI and two DNA fragments representing 93% of the Z gene were isolated. The termini of each fragment were different from any other termini used in this construction. The isolated fragments were 2115 bp (hereinafter referred to as B2) and 737 bp (herein after referred to as B3). The EcoRI restriction site in the Z gene is near the C-terminal end of the gene. This terminus must be present when the Z gene containing an XhoI site is constructed.

The mutant Z gene was inserted in pF29. Plasmid pF29 contains a Z gene α-region fused to the C-terminal end of the Z gene at the EcoRI site. This α-region is controlled by the λPr promotor inserted at a BamHI site. To construct pF29 two intermediate plasmids, pF15 and pF16 were constructed. pβga12 was digested with AvaI and the cohesive 3' end filled in using the Klenow fragment and the four dNTPs to create blunt ends. A SalI linker (GGTCGACC) (New England BioLabs, Beverly, MA) was ligated to the linearized plasmid using T4 DNA ligase. The resultant DNA was digested with EcoRI and SalI, and a 300 bp DNA fragment representing the omega (ω) end of the β-qalactosidase Z gene purified by agarose gel electrophoresis. The ω-region was fused to an α-region under control of λPr as follows. pUC12 DNA (Bethesda Research Laboratories, Gaithersburg, MD) was digested with BglI and blunt ends created by treatment with Klenow fragment and the four dNTPs. EcoRI linkers (GGAATTCC) (New England BioLabs, Beverly, MASS) were ligated to the blunt ends with T4 DNA ligase. The DNA was digested with BamHI and EcoRI and a 180 bp fragment representing the α-region of the Z gene was purified by agarose gel electrophoresis. The vector used to accept the α- and ω-gene fragments was pβga12 digested with BamHI and SalI and purified by agarose gel electrophoresis to remove the lac operon sequences. The vector, α-gene and ω-gene fragments were ligated together using T4 DNA ligase. The unique ends of the DNA fragments direct the order in which these fragments were cloned. The product plasmid was designated pF15.

pF15 was further modified by converting the unique PvuII site into the vector SalI site using SalI linkers ligated to the blunt ends created by digesting pF15 with PvuII. This modified pF15 was then digested with BamHI and SalI, and the largest DNA fragment was purified by agarose gel electrophoresis which removes the α-ω-gene sequence and a DNA fragement located between the SalI site and the PvuII site. Unmodified pF15 was also digested with BamHI and SalI and the α-ω fragment purified. When the large fragment from the modified pF15 was ligated to the α-ω fragment, the plasmid pF16 was generated.

pF16 is about 1350 base pairs smaller than pF15 and has the effect of moving a unique NdeI site much closer to the SalI site. This maneuver eliminates the unnecessary DNA sequences from being carried through subsequent constructions.

To construct pF29, pF16 was digested with ClaI and NdeI and the 1400 bp DNA fragment encoding the λCI, λPr, and the α- and ω-regions of β-galactosidase was purified by agarose gel electrophoresis. pUC13 was digested with AccI and NdeI and the vector was purified by agarose gel electrophoresis. Since the AccI and ClaI restriction sites have identical cohesive ends and the NdeI restriction sites share identical termini, ligation of the DNA insert from pF16 and the pUC13 vector can occur only in one orientation. Ligation with T4 DNA ligase yielded pF29. pF29 contains one EcoRI site and no ClaI sites which was desirable since a second EcoRI and the ClaI site would have interfered with the construction of modified plasmids (e.g., p149 and subsequent analysis of the deletion mutants created from p150 described below).

pF29 was digested with BamHI and EcoRI, the intervening α-donor was removed and this vector was filled-in using B1X plus B2, plus B3 (B1X+B2+B3). The unique single-stranded end of each piece defines the order in which the pieces can be ligated together. The B1X, B2 and B3 fragments were ligated into the pF29 vector digested with BamHI and EcoRI described above, thus reconstructing a Z gene with an XhoI linker at bp 102 encoding amino acid 34 under λPr control. The resultant plasmid was designated p149.

To create a method for screening for the creation of viable enzyme-acceptors following digesting with XhoI and Ba131 digestion, a separate α-donor without the XhoI site was inserted into p149. An FnuDII digestion fragment from pUC13 containing the lacZ operator, promotor and α-donor was inserted into the SalI site of p149 which had been filled-in with Klenow fragment. The resultant plasmid was designated p150. Deletions were created by digesting p150 with XhoI and then digesting the DNA with Bal 31 exonuclease. After Ba131 treatment, the plasmid was ligated with T4 DNA ligase and transformed into AMA1004 host cells (AMA1004 is galU, galK, strA^{r}, hsdR⁻ leuB6, trpC 9830, A(1aCIPOZ) C29, (Casadaban et al, 1983, Methods in Enzymology, 100:293), and screened on Luria-Bertani plates containing the inducer isopropylthiogalactoside (IPTG) and the chromogenic substrate 5-bromo-4-chloro-3-indoyl-β-D-galactopyranoside (Xgal, Sigma Chemical Co., St. Louis, MO). Colonies that were white at 30°C indicated creation of viable enzyme-acceptors. Colonies were selected and plasmid DNAs prepared. Plasmid DNAs were digested with SalI, to remove the α-donor, religated and transformed into AMA1004 host cells. The sequence deletions were confirmed by Maxam and Gilbert sequencing and the enzyme-acceptor proteins purified as described below. The resultant strains are shown in FIG. 5.

Enzyme-acceptors have been constructed utilizing DNA synthesis techniques. For example, enzyme-acceptor 1 (EA1) was constructed from pl49 except that the α-region which contains the XhoI linker was replaced with the following synthesized DNA fragments (5'→3');

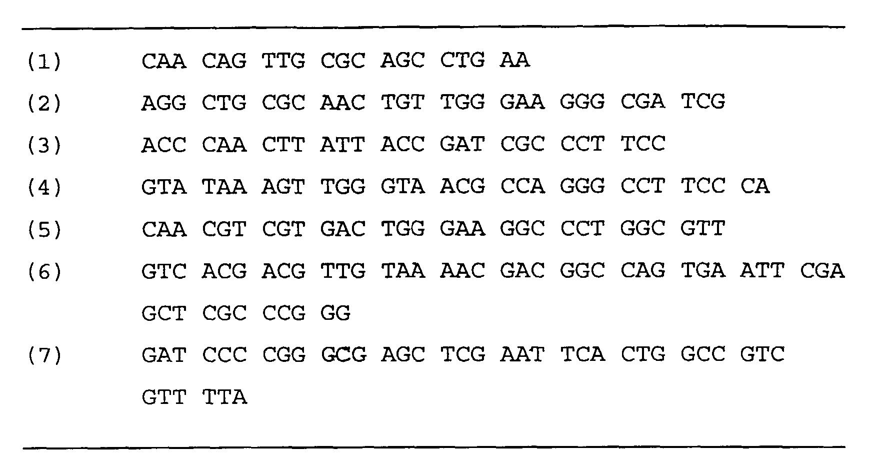

These fragments encode an in-frame deletion of amino acids 26-43 of the lac Z gene and carry BamHI and BglI sticky ends. These fragments were annealed, purified by gel electrophoresis, treated with BamHI and ligated to B2 plus B3 and the pF29 vector. A positive colony was selected and confirmed by DNA sequence analysis.

In accordance with the aforementioned fragments, analytes may be determined by forming a reaction mixture by combining in a medium (1) the sample; (2) an enzyme-donor polypeptide conjugate; (3) an analyte-binding protein specific for said analyte; and (4) an enzyme-acceptor polypeptide, consisting essentially of a fragment of β-galactosidase. The enzyme-donor polypeptide is characterized by forming with said enzyme-acceptor polypeptide, an active enzyme complex having β-galactosidase activity in the absence of analyte-binding protein binding to said conjugate. The enzyme-donor polypeptide conjugate is further characterized as being the N-terminal fragment of β-galactosidase with mutations as set out in claim 1.

In addition, a substrate capable of reacting with the active enzyme complexes is included in the medium, such that the rate of conversion by the active enzyme-complex can be monitored. The enzyme-donor conjugate is further characterized by being capable of competitively binding to the analyte-binding protein which results in inhibiting the formation of the active enzyme complex. After the reaction mixture is formed by combining the above components, the rate of conversion of substrate in the reacation mixture is measured and the amount of analyte is determined by comparing the rate of conversion of substrate in the sample containing medium with the rate of conversion of substrate obtained using a known concentration of analyte.

Of particular interest in the method are enzyme-donor compositions having one cysteine at amino acid positions 2, 20, 40 or 46, based on the β-galactosidase numbering as in FIG. 15. Alternatively, one cysteine may be in the N- or C- terminal sequence, where the N-terminal sequence is up to and including 27 amino acids and the C-terminal sequence is up to and including 17 amino acids.

Of particular interest is an enzyme-donor polypeptide which has an N-terminal sequence as Follows: 
M D P S G N P Y G I D P T E S S P G N I D P R A
S S N,

M D P S G D P R A S S N,

M D P R A S S N, or

C I T D;
 and a C-terminal sequence as follows:

A Q P E W G L E S R S A G M P L G,
R S L N G L E S R S A G M P L G, or
R S L N G E L C G V K Y R T D A.

### Comparison of Complementation Efficiency

In order to assess complementation efficiency of the enzyme-acceptors prepared as described above representative enzyme-acceptor preparations were compared using H6 as the enzyme-donor.

A microtiter plate format was used comprising a total volume of 200µl of PM2 buffer (0.5M Na₂HPO₄, pH7.0, 1 mM MgSO₄, 0.18 mM MnSO₄, 1 mM EDTA, 0.02% NaN₃, 0.05% Tween™ 20) containing 2.5X10⁻⁸M of the appropriate enzyme-acceptor preparation and 1.25 mg/ml o-nitrophenol-β-galactopyranoside substrate. A series of dilutions of H6 (1:20; 1:40; 1:80) were added to initiate complementation. The optical density (414 nm) was measured at 30 and 45 minutes incubation at 37°C. The results are illustrated in Table II.

**TABLE II**

| H6 Dilution | EA23 | EA14 | EA22 | EA24 | EA20 |
|---|---|---|---|---|---|
| A. OD₄₁₄ After 30 Minutes Incubation at 37°C | | | | | |
| 1/20 | .118 | .736 | .708 | .273 | .526 |
| 1/40 | .062 | .351 | .361 | .142 | .277 |
| 1/80 | .030 | .171 | .174 | .071 | .128 |

| B. OD₄₁₄ After 45 Minutes Incubation at 37°C | | | | | |
|---|---|---|---|---|---|
| 1/20 | .299 | 1.585 | 1.402 | .579 | 1.148 |
| 1/40 | .154 | .776 | .715 | .299 | .610 |
| 1/80 | .068 | .365 | .345 | .147 | .294 |

As demonstrated in Table I, the complementation efficiency of the various enzyme-acceptors varied considerably. The relative complementation efficiencies were:
EA14=EA22>EA20>EA24>EA23.

### EXAMPLE: ENZYME IMMUNOASSAY FOR DIGOXIN

This example illustrates an enzyme immunoassay suitable for use in the present invention where the analyte is the cardiotonic digitalis glycoside digoxin. The analyte-binding protein is an antibody specific for digoxin. The example further demonstrates that the mechanism of action of the assay is not analogous to the steric hinderance enzyme immunoassay using β-galactosidase described by Castro and Monji (1981, Methods in Enzymology 73:523-42).

### Preparation of Digoxin-ED₄ Conjugate

A urethane derivative of digoxigenin specifically 3-O-[m-maleimidophenylcarbamyl] digoxigenin [hereinafter termed "digoxin-malemide adduct"] was prepared as follows:

To a dry 10 ml round bottom flask equipped with a magnetic stirring device, an argon inlet, and a reflux condenser, was added 3-carboxylphenylmaleimide (67 mg or 0.307 mmole), dry benzene (3 ml), and dry triethylamine (0.043 ml or 0.307 mmole). The mixture was refluxed for 30 minutes. An infrared spectra analysis (IR) of an aliquot showed conversion to carbonyl azide (2150 cm⁻¹). Digoxigenin (80 mg or 0.205 mmole) and dry tetrahydrofuran (2 ml) were then added to the reaction mixture. After 3.5 hours of refluxing, the reaction mixture was diluted with ethyl acetate (100 ml), washed once with 50 ml cold 1% aqueous NaOH, and once with 50 ml saturated aqueous NaHCO₃. The organic layer was then dried over anhydrous MgSO₄, filtered, and the solvent removed by rotary evaporation. The residue was dissolved in approximately 1-2 ml acetone and applied to two preparative thin layer chromatography (TLC) plates (1500 micron silica gel Analtech uniplate, Analtech, Newarl, DE). When the acetone evaporated, the plates were eluted with 80/20 ethyl acetate/benzene. Unreacted digoxigenin was removed from the plate by washing it 3 times with 30 ml of ethyl acetate. This process was repeated for the next two spots above digoxigenin. This purification afforded digoxigenin (26 mg), the desired product digoxin-maleimide adduct (31 mg or 37% yield based on unreacted starting material), and ³⁻0-(m-maleimido-phenylcarbamyl)-digoxigenin (28 mg or 33% yield based on reacted starting material).

Thin layer chromatography was performed in 2.5% MeOH-CH₂Cl₂ to ascertain the purity of the digoxin-maleimide adduct. If further purification is desired, this is best accomplished by preparative TLC with 3% MeOH/CH₂Cl₂ (2 elutions). The digoxin-maleimide adduct had the following spectral characteristics: Infrared (nujol mull): 3490, 3350, 1805, 1760, 1725, 1700, 1615, 1550, 1460, 1305, 1240, 1160, 960, 935, 905, 880, 840, 810, 790, 710 cm⁻¹. (NMR, Nuclear Magnetic resonance acetone d₆): 0.8 (3 H,s), 3.38 (1 H, brs), 3.40 (1H, q, J=4.78 Hz), 4.84 (2 H, t, J=1.5 Hz), 5.00 (1H, m), 5.78 (1H, t, J=1.5 Hz), 6.98 (s, 2 HO), 6.8-7.7 (4 H, m), 8.75 (1 H, br s). Mass spectrum (CDI-NH₃): 622 (M+NH₄+) 605 (M+H³⁰) 587 (M+H+-H₂), 391, 373, 355, 337, 214, 191, 189.

The digoxin-maleimide adduct was then further purified on a RP-8 SynChropak 250 X 10 mm I.D. (SynChrom, Inc., Linden, ID) using a Beckman Model 332 high performance liquid chromatography system (Beckman Instruments, Inc., Palo Alto, CA). Gradient elution was performed from 0-80% acetonitrile in H₂O over 60 minutes at a 1.5 ml/minute flow rate. The digoxin-maleimide adduct was pooled and lyophilized.

The purified digoxin-maleimide adduct was then coupled to the enzyme-donor ED₄, prepared as described supra, to form digoxin-ED₄, an enzyme-donor analyte conjugate. ED₄ (1.5 mg) was dissolved in 240 µl acetonitrile-50 mM sodium phosphate (3:2) at pH 6.0. Digoxin-maleimide adduct (1.0 mg) was added directly to the reaction mixture which was maintained at 37°C for two hours. Upon completion of the coupling reaction, 60 µl aliquots of the mixture were injected onto a Bondapak® Phenyl column 10 X 30 cm (Waters Associates, Milford, MA). The column was developed with a 60 minute gradient 0-80% acetonitrile in H₂O, 0.1% trifluoroacetic acid. Samples containing enzyme-donor activity were pooled.

### Immunoassay For Digoxin

In enzyme immunoassay systems prepared according to the methods of the present invention, varying combinations of concentrations of enzyme-acceptor and enzyme-donor conjugate (i.e., enzyme-donor coupled to analyte) can be used to produce a given β-galactosidase concentration via the complementation process. The law of mass action requires that at relatively high concentrations of enzyme-acceptor, the inhibitory influence of the antibody on the complementation process is mitigated. This is evidenced by flat or absent dose-response characteristics with varying concentrations of analyte, e.g., digoxin. Conversely, at relatively high concentrations of enzyme-donor conjugate (compared to antibody), the inhibitory influence of the antibody on the complementation process is also lost. The latter situation is also evidenced by flat or absent dose-response characteristics and an elevated background.

This example illustrates that just as in conventional enzyme immunoassays, the relative concentrations of enzyme-acceptor, enzyme-donor and specific antibody must be defined to produce an assay with dose-response characteristics having suitable precision (slope) and sensitivity for use in a diagnostic assay for analyte.

In one series of experiments using a microtiter format, the sensitivity of the system was determined using different combinations of digoxin-ED₄ enzyme-donor and EA23 enzyme-acceptor concentrations.

Assays were performed by adding four sequential additions of 50 µl each, digoxin (analyte), enzyme-donor H6 digoxin conjugate, antibody specific for digoxin (anti-digoxin) and solution containing both enzyme-acceptor (EA23) and o-nitrophenyl-β-D-galactopyranoside (ONPG) 5 mg/l as substrate. All dilutions were performed in PM2 Buffer [0.5M Na₂HPO₄, 1 mM MgSO₄, 1 mM EDTA, 0.02% NaN₃ and 0.05% Tweed™ 20 (polyoxyethylene sorbitan monolaurate, Sigma Chemical Co., St. Louis, MO). The concentrations of the digoxin analyte were: 0, 1, 10, 100, 200, 500 and 1000 ng/ml. Antibody specific for digoxin was obtained by injection of digoxin conjugate into rabbits as follows: Primary intramuscular injections were made using 50 µl of conjugate in a total volume of 2.0 ml complete Freund's adjuvant. Boosters (intramuscular) were administered at 4-week intervals with 25 µg of conjugate in a volume of 1.0 ml complete Freund's adjuvant. 50 ml bleeds were collected every two weeks starting 90 days following primary injection. Collections were made by phlebotomy of the medial artery or lancing of the marginal ear veins. Blood was allowed to coagulate and 25 ml serum/50 ml blood recovered as supernatant following 30 minutes centrifugation at 1000 X g. The results were graphed. A comparison of the dose-response curves in showed that selective reduction of the concentration of either enzyme-acceptor or enzyme-donor conjugate produces a steeper, and hence more sensitive dose-response curve.

### Mechanism of Digoxin Immunoassay

In order to determine whether reacting the anti-digoxin antibody with the enzyme-donor digoxin conjugate was interfering with the complementation process rather than with conversion of substrate by polymerized β-galactosidase enzyme, the complementation process was allowed to proceed to completion prior to addition of antibody in one series of experiments.

The protocol of the experiments was as follows: 300 µl of PM2 Buffer and the digoxin-H6 conjugate were reacted for 60 minutes with 150 µl of the enzyme-acceptor EA23 (4.1 X 10⁻⁶M). This permitted the complementation to proceed to completion. An aliquot (125 µl) of the above reaction mixture was removed and added to an aliquot (50 µl) of rabbit anti-digoxin antibody (diluted 1:100 with PM2 Buffer). The reaction mixture was then incubated for 30 minutes. At the end of this time period, the ONPG substrate (final concentration 1 mg/ml) was added and the reaction mixture incubated at 37°C. The optical density of the reaction mixture was determined at 7 and 16 minutes following incubation at 37°C. Control tubes were treated similarly except that 50 µl of either normal rabbit serum diluted 1:100 with (PM2 Buffer) or PM2 Buffer was added instead of rabbit anti-digoxin antiserum. Results are illustrated in Table V.

**TABLE V**

| | Optical Density Incubation Time | |
|---|---|---|
| Sample | 7 Minutes | 16 Minutes |
| Anti-digoxin^{(a)} | .475 | .947 |
| NRS^{(b)} | .466 | .954 |
| PM2^{(c)} | .457 | .936 |
| Substrate Blank | .050 | .057 |

| | | |
|---|---|---|
| (a) Anti-digoxin designates rabbit 539 (50 µl, 1:100 dilution in PM2 Buffer). | | |
| (b) NRS designates normal rabbit serum (50 µl 1:100 dilution in PM2 Buffer). | | |
| (c) PM2 Buffer: 0.5 M Na₂HPO₄, pH 7.0, 1 mM MgSO₄, 0,18 mM MnSO₄, 1 mM EDTA, 0.02% NaN₃, 0.05% Tween™ 20). | | |

As demonstrated in Table V, antibody did not inhibit conversion of substrate by the previously polymerized β-galactosidase (complete complementation of Digoxin-ED₄ and EA23 enzyme-acceptor). Thus, the decreased substrate conversion observed using the enzyme assay is the result of antibody-inhibited complementation, nor reduced enzyme substrate conversion. Therefore, the mechanism of action of the assay of the present invention is not analogous to the steric hinderance enzyme immunoassay using β-galactosidase described by Castro and Monji (1981, Methods in Enzymology 73:523-542).

### Effect of Anti-Digoxin Antibody on Complementation Using a Variety of Enzyme-Acceptors

In one series of experiments, the inhibitory effect of specific antibody against digoxin was determined using three enzyme-acceptors and the enzyme-donor digoxin-H6 conjugate prepared as described above.

The reaction mixture was prepared as follows: 50 µl PM2 Buffer, 50 µl of the appropriate dilution (1:20, 1:40, 1:80) of digoxin-ED₄ conjugate in PM2 Buffer; 50 µl of the appropriate antibody (i.e., either anti-digoxin antibody to normal rabbit serum) and 50 µl of the appropriate mixture of enzyme-acceptor (1 X 10⁻⁷ EA14, EA20 or EA22) and substrate o-nitrophenol-β-D-galactopyranoside (ONPG) (5 mg/ml) were added to a microtiter plate. The plates were then incubated at 37°C for specified time periods. The optical density at 414 nm was determined at 5 minute intervals for 45 minutes.

The following table indicates α-region enzyme acceptor sequences.

*                   10                  20
M D P R A S S N S L A V V L Q R R D W E N P G V T E

          30                  40
L N R L A A H P P F A S W R N S E E S R T D R P S Q

50                  60
Q L R S L N G E L R F

| | Amino Acid Deletions |
|---|---|
| M15 | 11 - 41 |
| M112 | 23 - 31 |
| EA5 | 35 - 52 |
| EA11 | 35 - 54 |
| EA14 | 30 - 37 |
| EA17 | 21 - 53 |
| EA18 | 13 - 45 |
| EA20 | 126 - 45 |
| EA22 | 13 - 40 |
| EA23 | 16 - 35 |
| EA24 | 22 - 35 |

The inhibitory effect of antibody on complementation in this system appears to relate to the size of the deletion in the enzyme-acceptor. Enzyme-acceptor EA22 which deletes amino acids 13-40 and is the largest deletion tested in this experiment was inhibited least by antibody. Enzyme-acceptor, EA14 which deletes amino acids 30-37 is the smallest of the tested group and was inhibited the most by antibody. EA 20 which comprises amino acids 26-45 and is intermediate in size between EA22 and EA14 was relatively moderately inhibited. The native complementation efficiency of EA20 is, however, lower than that of either EA14 or EA22. The enzyme-acceptor must satisfy two criteria: (a) native complementation efficiency e.g., EA14 and EA22 are more efficient than other sequences based on equimolar concentrations; and (b) the ability of specific analyte-binding protein to inhibit complementation.

### EXAMPLE: EFFECT OF A SECOND ANTIBODY ON THE DIGOXIN ENZYME IMMUNOASSAY

The results presented above suggest that coupling of anti-digoxin antibody to the enzyme-donordigoxin conjugates of the present invention slows down the rate of complementation of enzyme-acceptor and enzyme-donor conjugate. Such coupling, however, does not completely prevent complementation. Thus, as stated in above the system has greatest sensitivity at approximately 15 minutes incubation of enzyme-acceptor and enzyme-donor conjugate.

The system reaches maximum absorbance differential at approximately 15 minutes. At that time the concentration of β-galactosidase in a system with anti-digoxin antibody present is the same as that in a system in which the antibody is absent, or in which digoxin antibody is neutralized (e.g., high digoxin levels). Since the β-galactosidase concentration is the same, the rate of substrate conversion is the same. No additional absorbance differential occurs. This phenomenon, which limits the effectiveness of the antibody on complementation produces a narrow absorbance range for a dose-response curve, flat slope characteristics and inadequate sensitivity of the assay for some diagnostic applications.

The following example demonstrates that attachment of a secondary antibody, specific for the anti-digoxin conjugate antibody, enhances the inhibition of complementation.

### Attachment of Whole Secondary Antibody

In one series of experiments, 50 µl of rabbit anti-digoxin antibody (diluted 1:1000) was combined in a set of microtiter wells with 50 µl of digoxin-H6 (diluted 1:50 in PM2 buffer), and 50 µl of digoxin, in concentration of 0, 1, 2.5, 5, 7.5, 10, 100 ng/ml. A 50µl aliquot of a secondary antibody preparation (Bethyl Lab, Montgomery, TX, goat anti-rabbit serum 1:50-1:800) was added to each well. Results are tabulated in Tables VI and VII.

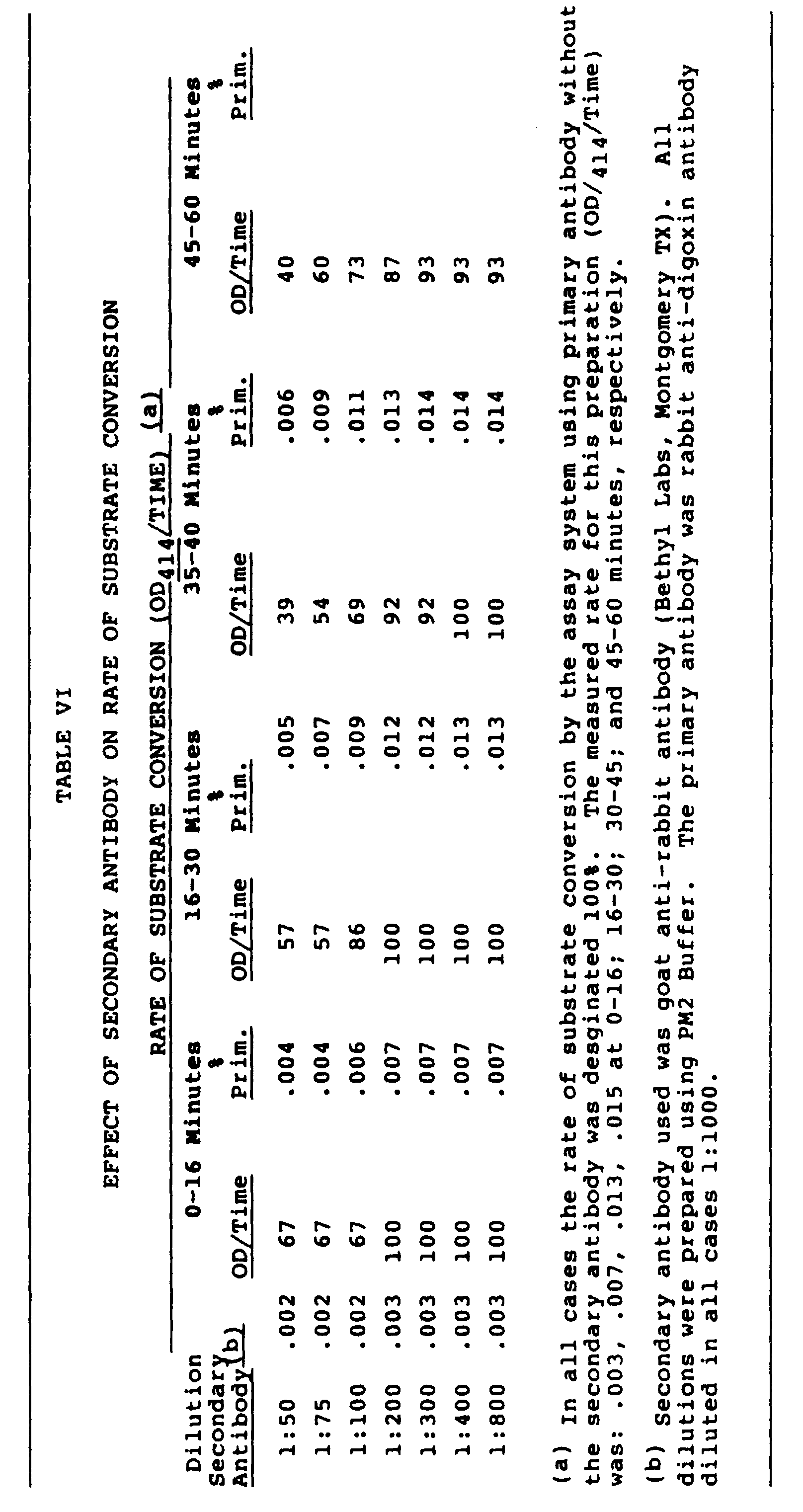

As demonstrated in Table VI, the inhibitory effect on complementation achieved by attaching a secondary antibody to the antibody-digoxin H6 conjugate is optimal at a 1:50 dilution or less of the secondary antibody. At a dilution of 1:200 to 1:300 of the secondary antibody, all synergistic inhibition is lost. Thus, inhibition of complementation with or without secondary antibody is the same at this dilution or greater.

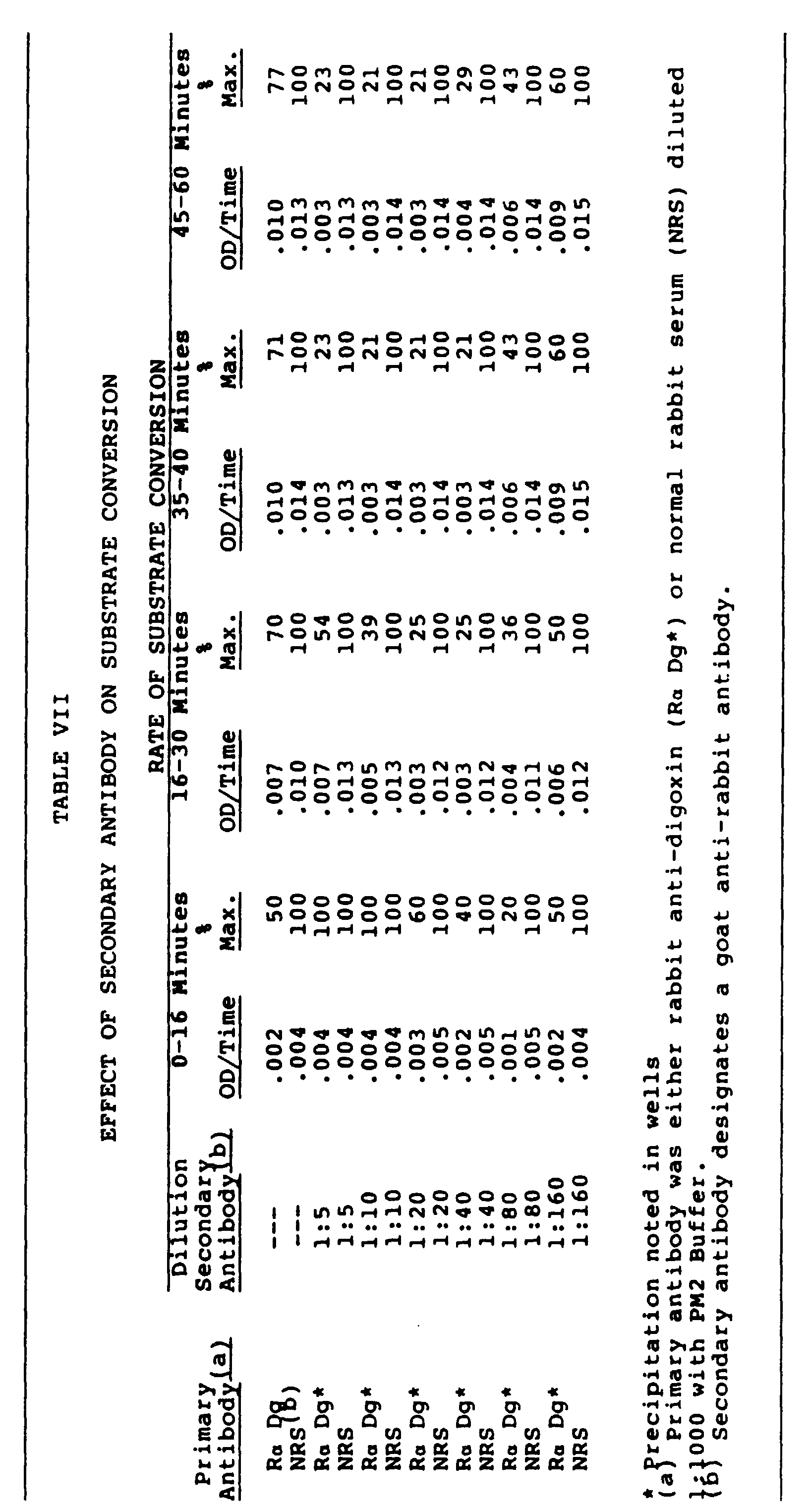

As demonstrated in Table VII, with no secondary antibody, the rate of substrate conversion (i.e., β-galactosidase concentration) reached 70% of maximum within 30 minutes. With secondary antibody at a 1:40 dilution, the rate of substrate conversion was approximately 25% of maximum. At greater than 1:40 dilutions of secondary antibody, the inhibitory effect on complementation diminished as evidenced by increasing rates of substrate conversion over time.

At dilutions equal to or greater than 1:40, the effect is an increase in the rate of substrate conversion.

At all concentrations of secondary antibody tested, the rate of substrate conversion (i.e., β-galactosidase concentration) became linear (i.e., no new β-galactosidase produced) at levels below the maximum concentration of β-galactosidase the system would permit (e.g., NRS replaces secondary antibody). This indicates that binding of secondary antibody enhances steric interference of the primary antibody and may completely prevent complementation by that enzyme-donor population which is bound.

### Attachment of Fragment of Secondary Antibody

In order to determine the enhanced inhibition, observed when a secondary antibody was coupled to the primary antibody-enzyme-donor conjugate could be attributed to steric hinderance or entrapment of enzyme-donor conjugate in a precipitin complex, monovalent Fab fragments (antigen binding fragments about 50,000 daltons MW) of goat anti-rabbit immunoglobulin were used as the secondary antibody. Because Fab fragments cannot cross link antigen they are not capable of inducing a precipitin or an agglutination reaction. Any inhibition of complementation observed in this preparation is due to enhanced steric effects on complementation and not to enhanced entrapment of conjugate.

In a microtiter plate format, five equal additions of 50 µl each sequentially of digoxin (0, 1, 4, 10, 1000 ng/ml); digoxin-H6 conjugate; rabbit anti-digoxin primary antibody (1:4000) and secondary goat anti-rabbit antibody (Bethyl Labs, Montgomery, TX) at 1:80 dilution. All dilutions were in PM2 Buffer. The secondary antibody was replaced by normal rabbit serum (1:80) and the Fab fragment of goat anti-rabbit serum (Cappel Laboratories, West Chester, PA) at dilutions of 1:10, 1:20, 1:40, 1:80, 1:160, 1:320. After 10 minutes at room temperature 50 µl of 1X10⁻⁶M EA14 and 5 mg/ml of the substrate ONPG were added and incubations continued 30 minutes at 37°C. OD₄₁₄ was measured and Bound/Maximum Boun (B/Bma) determined.

The results are demonstrated in Table VIII.

**TABLE VIII**

| EFFECT OF FAB FRAGMENTS | | | | | |
|---|---|---|---|---|---|
| Dilution of Secondary Antibody Preparation^{(a)} | Rate of Substrate Conversion (B/B MAX) Concentration Digoxin (ng/ml) | | | | |
| | 0 | 1 | 4 | 10 | 1000 |
| Goat anti-rabbit IgG^{(b)} | 56.7 | 66.3 | 81.9 | 94.0 | 100 |
| None^{(c)} | 95.4 | 91.3 | 88.7 | 97.1 | 100 |
| Fab 1:10 Goat anti-rabbit IgG^{(d)} | 62.8 | 68.1 | 75.2 | 89.4 | 100 |
| Fab 1:20 Goat anti-rabbit IgG^{(d)} | 57.6 | 67.6 | 75.5 | 87.1 | 100 |
| Fab 1:40 Goat anti-rabbit IgG^{(d)} | 66.2 | 73.8 | 80.0 | 90.3 | 100 |
| Fab 1:80 Goat anti-rabbit IgG^{(d)} | 69.7 | 75.6 | 82.3 | 91.5 | 100 |
| Fab 1:160 Goat anti-rabbit IgG^{(d)} | 77.4 | 78.5 | 83.1 | 92.11 | 100 |
| Fab 1:320 Goat anti-rabbit IgG^{(d)} | 81.6 | 83.5 | 85.1 | 94.8 | 100 |

| | | | | | |
|---|---|---|---|---|---|
| ^{(a)} All secondary antibody preparations were tested using primary antibody at 1:4000 dilution. | | | | | |
| ^{(b)} Goat anti-rabbit immunoglobulin antiserum (Bethyl Labs, Montgomery, TX). | | | | | |
| ^{(c)} None designates that no secondary antibody was used. A 1:80 dilution of normal rabbit serum replaced the secondary antibody in these samples. | | | | | |
| ^{(d)} Fab Goat anti-rabbit IgG designates the Fab fragment obtained from H and L Sp. (Cappel 0412-0081 Lot #23167) (Cappel Laboratories, West Chester, PA). | | | | | |

As demonstrated in Table VIII, decreased complementation is evident when the goat anti-rabbit immunoglobulin (Fab fragment) is coupled to the primary antibody enzyme-donor conjugate. The inhibition of complementation induced by the Fab fragment is approximately equivalent to that inhibition observed when using whole antibody.

As shown in Table VIII, the secondary antibody had a greater inhibiting effect on complementation at low dose (i.e., greater antibody/enzyme-donor interaction be caused by excess free analyte).

Decreasing Fab concentration did produce a linear decline in complementation inhibition. In Table VI intact molecules demonstrated a decrease in secondary antibody effectiveness with dilution greater than 1:40. Likewise, the same phenomenon is seen beginning with 1:40 dilution of the Fab preparation.

### Comparison of Performance of Genetically Engineered and Chemically Synthesized Enzyme-Donors in Digoxin Immunoassay

To compare enzyme immunoassays performed with chemically synthesized versus genetically engineered components, two analogous enzyme-donors were prepared, one by recombinant DNA techniques and the other by chemical peptide synthesis. The amino acid sequences of ED3 created by genetic engineering and ED3A created by polypeptide synthesis are as follows: 
ED3
Asp Pro Ser Gly Asp Pro Arg Ala Cys Ser Asn Ser Leu Ala
Val Val Leu Gln Arg Arg Asp Trp Glu Asn Pro Gly Val Thr
Glu Leu Asn Arg Leu Ala Ala His Pro Pro Phe Ala Ser Trp
Arg Asn Ser Glu Glu Ala Arg Thr Asp Arg Pro Ser Gln Gln
Leu Arg Ser Leu Asn Gly Leu Glu Ser Arg Ser Ala Gly Met
Pro Leu Glu

ED3A
Cys Ile Thr Asp Ser Leu Ala Val Val Leu Gln Arg Arg Asp
Trp Glu Asn Pro Gly Val Thr Gln Leu Asn Arg Leu Ala Ala
His Pro Pro Phe Ala Ser Trp Arg Asn Ser Glu Glu Ala Arg
Thr
 The salient features of these two peptides are the analogous cysteine residue (Cys), marked with an asterisk used for chemical coupling to a analyte and the analogous α-donor domain, which in ED3 is located between amino acids number 12 and 50, inclusive, and in ED3A is located between amino acids number 5 and 43 inclusive, and correspond to amino acids 6 through 44 of wild-type-β-galactosidase.

Conjugation of digoxin to ED3 and ED3A was performed with 3-O-[maleimidophenylcarbamyl]-digoxigenin as described. Preparations of ED3, ED3A, digoxin-ED3 and digoxin-ED3A were subjected to high performance liquid chromatography (HPLC) on a preparative HPLC phenyl column (Waters µBondapak, Waters Assoc., Milford, MA) using a gradient of 0.80% acetonitrile in water containing 0.1% TFA as eluent. Column fractions of each enzyme-donor were assayed for complementation as described above using M15 as enzyme-acceptor. The relative complementation efficiency of ED3-digoxin was four times greater than ED3A-digoxin.

Column fractions corresponding to digoxin-ED3 and digoxin-ED3A were pooled separately and compared in a competitive enzyme immunoassay for digoxin.

A 96-well microtiter plate was used for the assay. The assay comprised 25 µl of human serum standards 0, 0.5, 1, 2, 4, 10, 100 and 1000 ng/ml digoxin, 100 µl of reagent I which contains 4 X 10⁻⁷M M15 enzyme-acceptor and digoxin antibody, and 130 µl of reagent II. Reagent II contained various dilutions of digoxin-ED3 or digoxin-ED3A, secondary goat anti-rabbit antibody and 1.1 mg/ml of o-nitrophenyl-β-D-galatopyranoside. The results following a 30 minute incubation at 37°C and reading at 405 nm in a Titertek microtiter plate reader are shown in Table X, competitive immunoassays were created with both digoxin-ED3 and digoxin-ED3A. The digoxin immunoassay with digoxin-ED3 gave better signal discrimination at the low doses of 0.5 and 1 ng/ml than digoxin-ED3A. This discrepancy may be due to the presence of impurities in the ED3A preparation which were detected during HPLC analysis.

These experiments demonstrate the applicability of synthesized polypeptides, as well as genetically engineered polypeptides, in the control of the complementation of β-galactosidase polypeptides by antigen-antibody reaction. Hence, chemical polypeptide synthesis can be used to create enzyme-donors for the purpose of detecting high molecular weight proteins. Gene fusions that encode immunologically reactive polypeptide epitopes fused to the α-donor domain can also be synthesized. The limits on this approach include not only the state-of-the-art capability to synthesize ever larger polypeptides but also knowledge of the sequence of both the required α-donor domain and the immunologically reactive protein domain.

**TABLE X**

| DIGOXIN ASSAY WITH ED3 AND ED3A | | | | | | | | |
|---|---|---|---|---|---|---|---|---|
| Conjugate Dilution | Digoxin Dose ng/ml | | | | | | | |
| | 0 | 0.5 | 1 | 2 | 4 | 10 | 100 | 1K |
| ED3 (Absolute OD) | | | | | | | | |
| 1/100 | .810 | .821 | .855 | .916 | .980 | 1.088 | 1.159 | 1.218 |
| 1/200 | .350 | .368 | .386 | .420 | .444 | .508 | .566 | .586 |
| 1/400 | .154 | .149 | .163 | .178 | .189 | .230 | .249 | .264 |
| 1/800 | .080 | .078 | .084 | .095 | .090 | .114 | .133 | .121 |

| ED3A (Absolute OD) | | | | | | | | |
|---|---|---|---|---|---|---|---|---|
| 1/100 | .668 | .656 | .660 | .660 | .668 | .719 | .757 | .777 |
| 1/200 | .310 | .306 | .309 | .322 | .329 | .352 | .372 | .375 |
| 1/400 | .146 | .146 | .153 | /152 | .151 | .192 | .180 | .180 |
| 1/800 | .043 | .039 | .047 | .043 | .048 | .060 | .064 | .052 |

| ED3 (ΔOD) | | | | | | | | |
|---|---|---|---|---|---|---|---|---|
| 1/100 | 0 | .011 | .045 | .160 | .170 | .278 | .349 | .408 |
| 1/200 | 0 | .018 | .036 | .070 | .094 | .158 | .216 | .236 |
| 1/400 | 0 | .005 | .009 | .024 | .035 | .076 | .095 | .110 |
| 1/800 | 0 | .002 | .004 | .015 | .010 | .034 | .053 | .041 |

| ED3A (ΔOD) | | | | | | | | |
|---|---|---|---|---|---|---|---|---|
| 1/100 | | -.012 | -.008 | -.008 | 0 | .051 | .089 | .104 |
| 1/200 | | -.004 | -.001 | -.012 | .019 | .042 | .062 | .065 |
| 1/400 | | 0 | .007 | .006 | .005 | .046 | .034 | .034 |

A number of additional enzyme donor sequences were prepared, where the basic sequences as follows and the following table indicates the substitutions and deletions for the enzyme donor sequences.

1          5          10          15          20          25
M D P S G N P Y G I D P T Q S S P G N I D P R A S S N S

 30          35          40          45          50          55
L A V V L G R R D W E N P G V T Q L N R L A A H P P F A

     60          65          70          75          80
S W R N S E E A R T D R P S Q Q L R S L N G L E S R S A

85
G M P L G

The enzyme donor conjugates were characterized by determining the immunopurity of the conjugate; the kinetic specific activity; the percent inhibition at half saturation; the maximum amount of complementation inhibition and the affinity constant for antibody to the ligand. The assay conditions, except where otherwise indicated are as follows:

The reagents employed were sample buffer: 100 mM sodium phosphate, 20 mM sodium azide and 0.2% BSA; assay buffer pH 7.0: 10 mM ethylene glycol, tetraacetic acid, 2 mM Mg acetate, 20 mM sodium azide, 150 mM sodium phosphate, 100 mM potassium phosphate, 0.05% Tween™ 20, 0.05 mM dithiothreitol.

Immunopurity was determined by precipitation of the enzyme donor conjugate with anti-T4 antibody TgG Sorbheads. The ED is incubated with primary antibody at room temperature for 15 min on a rocker shaker. Secondary antibody is added and incubation continued for 15 min. The suspension is centrifuged and an aliquot of the supernatant is saved for the assay. The above cycle is repeated on the remaining supernatant for a total of five cycles. A control sample of the ED has no primary antibody. The ED concentration during incubation is 20 nM. Assay concentrations are EA 500 nM; ED, 4 nM; and ONPG, lmg/ml. Immunopurity is expressed as percent of ED activity precipitable by primary antibody.

The kinetic specific activity is determined using a COBAS BIO™ assay with pre-incubation of the ED with excess EA for 10 min. System concentrations are: ED 0.1-0.5 nM, EA 22, 500 nM and CPRG, 1.11 mM. Six ED concentrations in the above range were assayed in duplicate at 37°C. Data of units per assay versus ng protein/assay are plotted and a specific activity (u/mg) is calculated by least squares fitting. One unit is defined as 1 µmole CPRG/min.

The immunochemical testing was performed on the Encore. A two reagent format was employed, the first reagent contained the assay buffer, EA22 and ONPG, while the second reagent contained the ED, anti-T4 or anti-digoxin antibody and the concentrations were EA, 560 mM; ED, 2.4 nM; ONPG, 0.58 ng/ml. The ligand concentrations employed are indicated in the legend to the table. The ED and antibody were preincubated on the rotor for 15 min at 37°C. All antibody concentrations were assayed in duplicate. No correction was made for variations of total protein in the assays.

The Ka was determined as follows: To determine the affinity constant of an antibody for an experimental ED conjugate, an ED titration against a fixed antibody is carried out. Antibody binding to the conjugated ED is monitored by inhibition of formation of active enzyme as indicated by a decrease in chromogenic substrate turnover. The resulting data is plotted in a Scatchard format (Bound/Free vs. [Bound]). The slope of the line generated is equal to -K_{A} in L/mole.

The following table indicates the results.

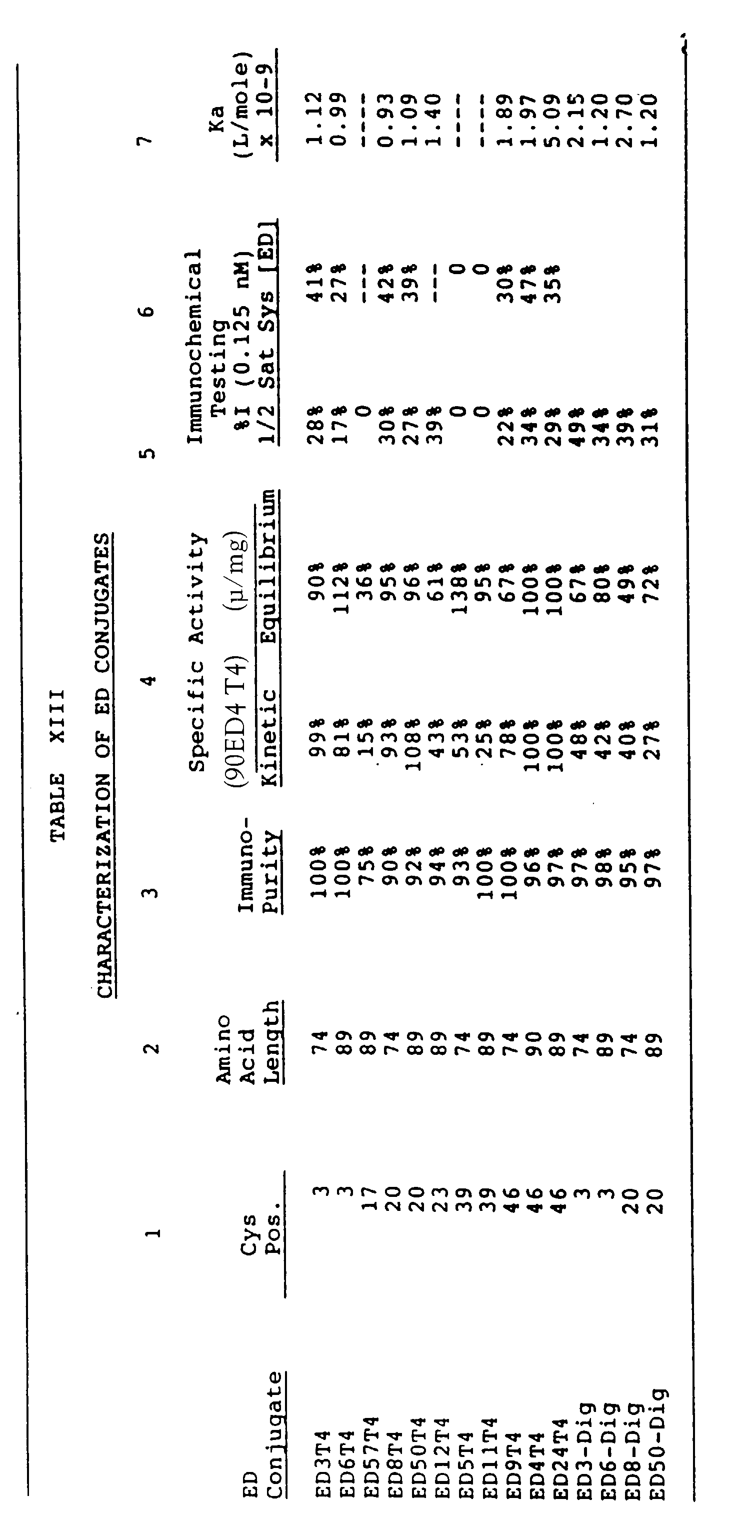

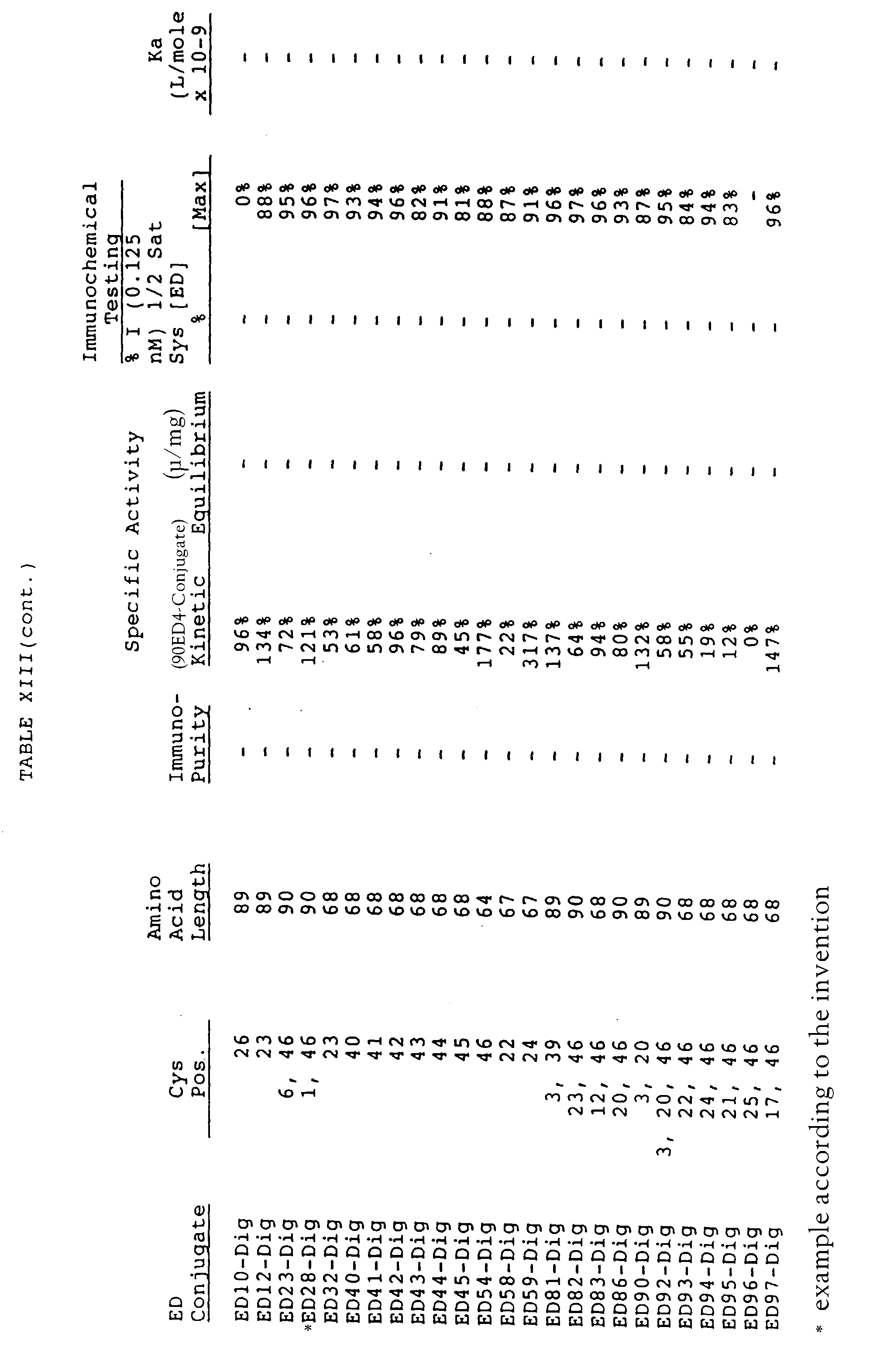

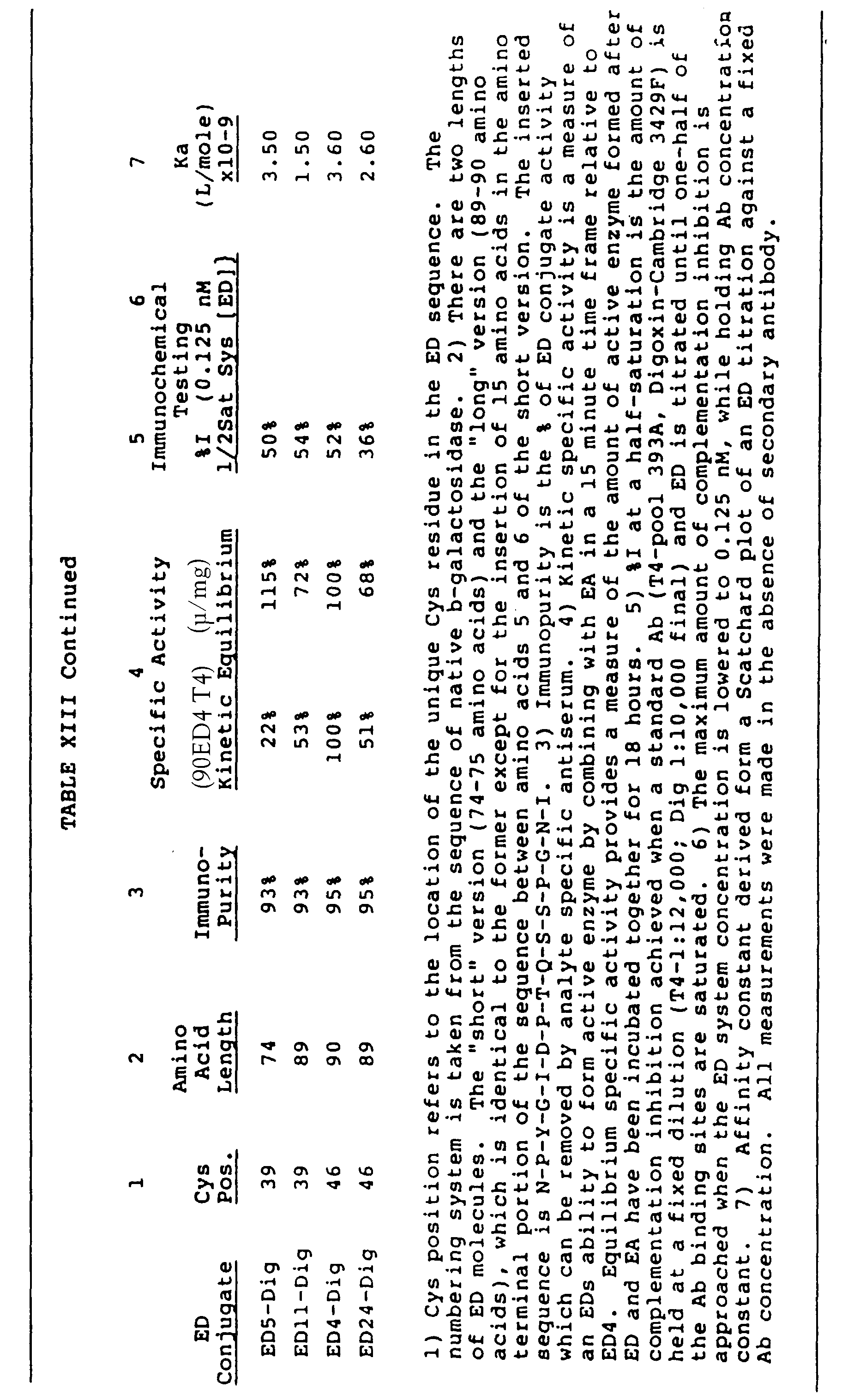

It is evident from the above results that the subject method provides for a sensitive and accurate assay, which may be employed in a variety of protocols. In addition, storage stability is greatly enhanced, since the fragments are quite stable and are readily activated upon combination in an aqueous medium. The individual fragments are easily conjugated to a wide variety of ligands of interest, to provide for active conjugates which result in a broad dynamic range for the various ligands.

Although the foregoing invention has been described in some detail by way of illustration and example for purposes of clarity of understanding, it will be readily apparent to those of ordinary skill in the art in light of the teachings of this invention that certain changes and modifications may be made thereto.

## Claims

1. An enzyme assay method for determining the amount of a suspected analyte in a sample, where said analyte is a member of a specific binding pair consisting of ligand and receptor, which method comprises the steps of:
(a) forming a reaction mixture by combining in a medium (1) sample; (2) an enzyme donor polypeptide comprising the N-proximal fragment of β-galactosidase; (3) an analyte binding protein, when said analyte is other than a receptor; and (4) an enzyme acceptor polypeptide consisting essentially of a C-proximal fragment of β-galactosidase, wherein said enzyme donor polypeptide is conjugated with a ligand cross-reactive with ligand analyte or complementary to receptor analyte, the enzyme donor β-galactosidase fragment being modified to provide a site for conjugation with said ligand, and said enzyme donor and said enzyme acceptor form an active enzyme complex having β-galactosidase activity, which activity is distinguishable when a receptor is bound to the conjugate of ligand and enzyme donor;
(b) measuring the rate of conversion of substrate in the reaction mixture; and
(c) determining the amount of analyte in the sample by comparing the rate of conversion of substrate to a rate of conversion of substrate obtained using a known amount of analyte,
characterized in that said enzyme donor β-galactosidase fragment is modified by the substitution of at least two amino acids two of the said substitutions being to cysteine or lysine and being at the 23 and 68 positions.

2. A method according to claim 1, wherein said substitution at the 23-position is to cysteine.

3. A method according to claim 1, wherein said analyte is a hapten.

4. A method according to claim 1, wherein said analyte is an antigen.

5. A method according to claim 1, wherein said reaction mixture further comprises an antibody that binds said receptor.

6. An enzyme assay method according to any one of the preceding claims, wherein the modified fragment is conjugated at a substitution site to a ligand.

7. A protein having at least 40 amino acids of the following sequence: 
1          5              10             15
M D P S G N P Y G I D P T Q S

          20        *    25             30
S P G N I D P R A S S N S L A

          35             40             45
V V L Q R R D W E N P G V T Q
                              20

          50             55             60
L N R L A A H P P F A S W R N

          65        *    70             75
S E E A R T D R P S Q Q L R S
 40                           50

          80             85          89
L N G L E S R S A G M P L G
 56
 wherein the numbers underneath letters indicate the wild-type β-galactosidase numbering and asterisks indicate sites for substitution of two different amino acids for conjugation to a ligand, said substitutions being to cysteine or lysine, and being at the 23 and 68 positions (1 and 46 wild-type positions).

8. A protein according to claim 7, wherein one of the substitutions is to cysteine.

9. A protein according to claim 7, wherein said sequence is joined to an amino acid sequence at either terminus defining an epitope of an antigen of interest.

10. A protein according to claim 7, wherein said sequence is covalently bonded to a ligand.

11. A protein according to claim 10, wherein said ligand is digoxin, thyroxine, a drug of abuse, a therapeutic drug, a steroid, or an immuno-logically cross-reactive compound thereof.

12. A recombinant DNA vector comprising a replication system functional in a microorganism host and an expression cassette comprising transcriptional and translational initiation and termination regulatory regions functional in said host, and an open reading frame encoding a protein composition according to claim 7.

13. A bacterium comprising a vector according to claim 12.

14. A bacterium according to claim 13, wherein said bacterium is E. coli.

15. A kit for use in an assay according to claim 1, comprising, for use as the ingredients of a reaction mixture, an enzyme donor polypeptide comprising the N-proximal fragment of β-galactosidase; an analyte binding protein, when said analyte is other than a receptor; and an enzyme acceptor polypeptide consisting essentially of a C-proximal fragment of β-galactosidase, wherein said enzyme donor polypeptide is conjugated with a ligand cross-reactive with ligand analyte or complementary to receptor analyte, the enzyme donor β-galactosidase fragment being modified to provide a site for conjugation with said ligand, and said enzyme donor and said enzyme acceptor form an active enzyme complex having β-galactosidase activity, which activity is distinguishable when a receptor is bound to said conjugate;
characterized in that the β-galactosidase fragment is modified by the substitution of at least two amino acids two of the said substitutions being to cysteine or lysine for conjugation to a ligand and being at the 23 and 68 positions.

16. A kit according to claim 15 for use in carrying out an assay according to claim 1, wherein said analyte is a member of a specific binding pair consisting of ligand and receptor, further comprising a β-galactosidase substrate capable of reacting with the active enzyme complex formed from enzyme-donor polypeptide and enzyme-acceptor polypeptide such that conversion of said substrate by active enzyme can be monitored.

## Patentansprüche

1. Enzymassayverfahren zur Bestimmung der Menge eines vermuteten Analyten in einer Probe, worin der Analyt ein Bestandteil eines aus einem Liganden und einem Rezeptor bestehenden spezifischen Bindungspaares ist, welches Verfahren die folgenden Schritte umfaßt:
(a) das Bilden eines Reaktionsgemisches durch Kombinieren (1) einer Probe; (2) eines Enzymdonor-Polypeptids, umfassend das N-proximale Fragment von β-Galactosidase; (3) eines Analyten-Bindeproteins, wenn der Analyt kein Rezeptor ist; und (4) eines Enzymakzeptor-Polypeptids, das im wesentlichen aus einem C-proximalen Fragment von β-Galactosidase besteht, in einem Medium, worin das Enzymdonor-Polypeptid mit einem Liganden konjugiert ist, der mit Ligandenanalyt kreuzreaktiv oder zu Rezeptoranalyt komplementär ist, wobei das Enzymdonor-β-Galactosidase-Fragment modifiziert ist, um eine Stelle zur Konjugation mit dem Liganden bereitzustellen, und der Enzymdonor und der Enzymakzeptor einen aktiven Enzymkomplex mit β-Galactosidase-Aktivität bilden, die unterscheidbar ist, wenn ein Rezeptor am Konjugat aus Ligand und Enzymdonors gebunden ist;
(b) das Messen der Umwandlungsrate von Substrat im Reaktionsgemisch; und
(c) das Bestimmen der Menge an Analyt in der Probe durch Vergleichen der Umwandlungsrate von Substrat mit der Umwandlungsrate von Substrat, die unter Verwendung einer bekannten Analytenmenge erhalten wird,
dadurch gekennzeichnet, daß das Enzymdonor-β-Galactosidasefragment durch Substitution von zumindest zwei Aminosäuren modifiziert wird, wobei zwei der Substitutionen zu Cystein oder Lysin erfolgen und an den Positionen 23 und 68 stattfinden.

2. Verfahren nach Anspruch 1, worin die Substitution an der Position 23 zu Cystein erfolgt.

3. Verfahren nach Anspruch 1, worin der Analyt ein Hapten ist.

4. Verfahren nach Anspruch 1, worin der Analyt ein Antigen ist.

5. Verfahren nach Anspruch 1, worin das Reaktionsgemisch weiters einen Antikörper umfaßt, der den Rezeptor bindet.

6. Enzymassayverfahren nach einem der vorhergehenden Ansprüche, worin das modifizierte Fragment an einer Substitutionsstelle mit einem Liganden konjugiert ist.

7. Protein mit zumindest 40 Aminosäuren der folgenden Sequenz: 
71

1 5 10 15
M D P S G N P Y G I D P T Q S

20 * 25 30
S P G N I D P R A S S N S L A

35 40 45
V V L Q R R D W E N P G V T Q
20

50 55 60
L N R L A A H P P F A S W R N

65 * 70 75
S E E A R T D R P S Q Q L R S
40 50

80 85 89
L N G L E S R S A G M P L G
56
 worin die Zahlen unter den Buchstaben die Numerierung der Wildtyp- β-Galactosidase und die Sterne die Substitutionsstellen zweier verschiedener Aminosäuren zur Konjugation mit einen Liganden kennzeichnen, wobei die Substitutionen zu Cystein oder Lysin erfolgen und an den Positionen 23 und 68 stattfinden (Wildtyp-Positionen 1 und 46).

8. Protein nach Anspruch 7, worin eine der Substitutionen zu Cystein erfolgt.

9. Protein nach Anspruch 7, worin die Sequenz an einem oder beiden Termini, mit einer Aminosäuresequenz verbunden ist, die ein Epitop eines Antigens von Interesse definiert.

10. Protein nach Anspruch 7, worin die Sequenz kovalent an einen Liganden gebunden ist.

11. Protein nach Anspruch 10, worin der Ligand Digoxin, Thyroxin, eine Mißbrauchdroge, ein Therapeutikum, ein Steroid oder eine immunologisch kreuzreaktive Verbindung davon ist.

12. Rekombinanter DNA-Vektor, umfassend ein Replikationssystem, das in einem Mikroorganismus-Wirt funktional ist, und eine Expressionskassette, die Transkriptions-, Translations-, initiations- und -terminationsregulationsbereiche umfaßt, die im Wirt funktional sind, und einen offenen Leserahmen, der für eine Proteinzusammensetzung nach Anspruch 7 kodiert.

13. Bakterium, umfassend einen Vektor nach Anspruch 12.

14. Bakterium nach Anspruch 13, worin das Bakterium E.coli ist.

15. Set zur Verwendung in einem Assay nach Anspruch 1, umfassend zur Verwendung als Ingredientien eines Reaktionsgemisches ein Enzymdonor-Polypeptid mit dem N-proximalen Fragment von β-Galactosidase; ein Analyt-Bindeprotein, wenn der Analyt kein Rezeptor ist; und ein Enzymakzeptor-Polypeptid, das im wesentlichen aus einem C-proximalen Fragment von β-Galactosidase besteht, worin das Enzymdonor-Polypeptid mit einem Liganden konjugiert ist, der mit Ligandenanalyt kreuzreaktiv oder zu Rezeptoranalyt komplementär ist, wobei das Enzymdonor-β-Galactosidasefragment modifiziert ist, um eine Stelle zur Konjugation mit dem Liganden bereitzustellen, und der Enzymdonor und der Enzymakzeptor einen aktiven Enzymkomplex mit β-Galactosidase-Aktivität bilden, die unterscheidbar ist, wenn ein Rezeptor am Konjugat gebunden ist;
dadurch gekennzeichnet, daß das β-Galactosidase-Fragment durch Substitution von zumindest zwei Aminosäuren modifiziert ist, wobei zwei der Substitutionen zu Cystein oder Lysin für die Konjugation mit einem Liganden erfolgen und an den Positionen 23 und 68 stattfinden.

16. Set nach Anspruch 15 zur Verwendung bei der Durchführung eines Assays nach Anspruch 1, worin der Analyt ein Bestandteil eines aus einem Liganden und einem Rezeptor bestehenden spezifischen Bindungspaares ist, weiters umfassend ein β-Galactosidase-Substrat, das mit dem aktiven Enzymkomplex reagieren kann, der aus Enzymdonor-Polypeptid und Enzymakzeptor-Polypeptid gebildet wird, sodaß die Umwandlung des Substrats durch aktives Enzym überwacht werden kann.

## Revendications

1. Méthode d'analyse d'enzyme pour déterminer la quantité d'un analyte suspecté dans un échantillon, où ledit analyte est un membre d'une paire de liaison spécifique consistant en un ligand et un récepteur, laquelle méthode comprend les étapes de :
(a) former un mélange réactionnel en combinant dans un milieu (1) l'échantillon ; (2) un polypeptide donneur d'enzyme comprenant le fragment N-proximal de la β-galactosidase ; (3) une protéine liant l'analyte, quand ledit analyte est autre qu'un récepteur ; et (4) un polypeptide accepteur d'enzyme consistant essentiellement en un fragment C-proximal de la β-galactosidase, où ledit polypeptide donneur d'enzyme est conjugué à un ligand réagissant mutuellement avec l'analyte ligand ou complémentaire de l'analyte récepteur, le fragment de β-galactosidase donneur d'enzyme étant modifié pour produire un site pour la conjugaison avec ledit ligand, et ledit donneur d'enzyme et ledit accepteur d'enzyme forment un complexe actif d'enzyme ayant une activité de β-galactosidase, laquelle activité peut être distinguée quand un récepteur est lié audit conjugué du ligand et du donneur d'enzyme ;
(b) mesurer le taux de conversion du substrat dans le mélange réactionnel ; et
(c) déterminer la quantité de l'analyte dans l'échantillon en comparant le taux de conversion du substrat à un taux de conversion du substrat obtenu en utilisant une quantité connue de l'analyte,
caractérisée en ce que ledit fragment de β-galactosidase donneur d'enzyme est modifié par la substitution d'au moins deux acides aminés, deux desdites substitutions étant à la cystéine ou la lysine et étant aux positions 23 et 68.

2. Méthode selon la revendication 1, où ladite autre substitution à la position 23 est à la cystéine.

3. Méthode selon la revendication 1, où ledit analyte est un haptène.

4. Méthode selon la revendication 1, où ledit analyte est un antigène.

5. Méthode selon la revendication 1, où ledit mélange réactionnel comprend de plus un anticorps qui lie ledit récepteur.

6. Méthode d'analyse d'enzyme selon l'une quelconque des revendications précédentes, où le fragment modifié est conjugué en un site de substitution à un ligand.

7. Protéine ayant au moins 40 acides aminés de la séquence suivante : 
1          5          10          15
M D P S G N P Y G I D P T Q S

          20     *     25          30
S P G N I D P R A S S N S L A

          35          40          45
V V L Q R R D W E N P G V T Q
                              20
          50          55          60
L N R L A A H P P F A S W R N

          65     *     70          75
S E E A R T D R P S Q Q L R S
40                         50
          80          85          89
L N G L E S R S A G M P L G
  56
 où les chiffres en dessous des lettres indiquent la numérotation de la β-galactosidase du type sauvage et les astérisques indiquent des sites pour la substitution de deux acides aminés différents pour une conjugaison à un ligand, lesdites substitutions étant à la cystéine ou à la lysine et étant aux positions 23 et 68 (positions 1 et 46 du type sauvage).

8. Protéine selon la revendication 7, où l'une des substitutions est à la cystéine.

9. Protéine selon la revendication 7, où ladite séquence est jointe à une séquence d'acides aminés à chaque terminal définissant un épitope d'un antigène d'intérêt.

10. Protéine selon la revendication 7, où ladite séquence est liée de manière covalente à un ligand.

11. Protéine selon la revendication 10, où ledit ligand et la digoxine, la thyroxine, une drogue d'usage abusif, un médicament thérapeutique, un stéroïde, ou un composé réagissant mutuellement immunologiquement de ceux-ci.

12. Vecteur d'ADN recombinant comprenant un système de réplication fonctionnel dans un micro-organisme hôte et une cassette d'expression comprenant les régions d'initiation de transcription et de traduction et de régulation de terminaison fonctionnelles dans ledit hôte et un cadre de lecture ouvert codant pour une composition de protéine selon la revendication 7.

13. Bactérie comprenant un vecteur selon la revendication 12.

14. Bactérie selon la revendication 13, où ladite bactérie est E. coli.

15. Nécessaire à utiliser dans une analyse selon la revendication 1, comprenant, pour une utilisation comme les ingrédients d'un mélange réactionnel, un polypeptide donneur d'enzyme comprenant le fragment N-proximal de la β-galactosidase ; une protéine liant l'analyte où ledit analyte est autre qu'un récepteur; et un polypeptide accepteur d'enzyme consistant essentiellement en un fragment C-proximal de la β-galactosidase, où ledit polypeptide donneur d'enzyme est conjugué à un ligand réagissant mutuellement avec l'analyte du ligand ou complémentaire de l'analyte du récepteur, le donneur d'enzyme, le fragment de β-galactosidase étant modifié pour reformer un site pour la conjugaison audit ligand, et ledit donneur d'enzyme et ledit accepteur d'enzyme forment un complexe d'enzyme actif ayant une activité de β-galactosidase, laquelle activité peut être distinguée quand un récepteur est lié audit conjugué ;
caractérisé en ce que le fragment de β-galactosidase est modifié par la substitution d'au moins deux acides aminés, deux desdites substitutions étant à la cystéine ou à la lysine pour une conjugaison à un ligand et étant aux positions 23 et 68.

16. Nécessaire selon la revendication 15 pour une utilisation pour effectuer une analyse selon la revendication 1, où ledit analyte est un membre d'une paire de liaison spécifique consistant en un ligand et un récepteur, comprenant de plus un substrat de β-galactosidase capable de réagir avec le complexe d'enzyme actif formé du polypeptide donneur d'enzyme et du polypeptide accepteur d'enzyme de façon que la conversion dudit substrat par l'enzyme active puisse être surveillée.
